(19) Europäisches Patentamt / European Patent Office / Office européen des brevets

(11) EP 1 605 981 B1

(12) # EUROPEAN PATENT SPECIFICATION

(45) Date of publication and mention of the grant of the patent: **08.04.2009 Bulletin 2009/15**

(21) Application number: **04713351.7**

(22) Date of filing: **20.02.2004**

(51) Int Cl.: ***A61L 15/44*** *(2006.01)* ***A61F 13/15*** *(2006.01)*

(86) International application number: **PCT/US2004/005083**

(87) International publication number: **WO 2004/073757 (02.09.2004 Gazette 2004/36)**

(54) **HEMORRHOID TREATMENT PAD**

VORLAGE ZUR BEHANDLUNG VON HÄMORRHOIDEN

TAMPON DE TRAITEMENT D'HEMORROIDES

(84) Designated Contracting States: **AT BE BG CH CY CZ DE DK EE ES FI FR GB GR HU IE IT LI LU MC NL PT RO SE SI SK TR**

(30) Priority: **20.02.2003 US 370748**

(43) Date of publication of application: **21.12.2005 Bulletin 2005/51**

(73) Proprietor: **THE PROCTER & GAMBLE COMPANY Cincinnati, Ohio 45202 (US)**

(72) Inventors:
- **WARREN, Raphael** Amberly Village, Ohio 45237 (US)
- **BLEVINS, John, Michael** Cincinnati, Ohio 45202 (US)
- **OSBORN, Thomas, Ward III** Ohio 45220 (US)

(74) Representative: **Kremer, Véronique Marie Joséphine et al Procter & Gamble Service GmbH Sulzbacher Strasse 40-50 65824 Schwalbach am Taunus (DE)**

(56) References cited:

**WO-A-99/45974** **WO-A-99/47116**
**WO-A-03/002698** **DE-A- 19 711 277**
**US-A- 4 040 424** **US-A- 5 695 484**
**US-A1- 2002 082 580**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 605 981 B1

## Description

### FIELD OF INVENTION

The present invention relates to absorbent anal pads. Specifically, this invention relates to hemorrhoid treatment pads intended to be worn by a person in need of hemorrhoid treatment.

### BACKGROUND OF THE INVENTION

External protruding hemorrhoids generally cause pain and discomfort. In addition, if the hemorrhoids become bruised, they may bleed which may cause the person alarm and concern. The use of hemorrhoid relief pads is known in the prior art. Pads have been proposed which employ belts and straps, but these pads can be uncomfortable and may be unsanitary if not disposable. Other pads have been proposed that, while avoiding the use of belts and straps, are highly invasive, requiring insertion through an often painfully-inflamed anus, and application of pressure to the affected area. Commercial emollient wipe treatments are available for insertion into the natal cleft. These treatments tend to be very oily and wet and can leave an undesirable residue on the user's hands requiring considerable washing and inconvenience.

U.S. Pat. No. 2,742,042 issued April 17, 1956 and RE 24,385 issued October 1, 1957 to Flanders discloses a napkin arranged as a pad for insertion into the anal declivity opposing the anus and held in place by the gripping action of the skin surfaces in the declivity. One potential problem with this approach is that the surface of the napkin abrasively contacts the skin surfaces of the natal cleft, which can irritate the rectal perineal body area causing chafing and related skin disorders. Another problem is that body exudates can provide an adhesive force between the napkin and the skin causing considerable discomfort, including tissue shearing, when the napkin is removed. There may also be unsatisfactory positioning of the napkin in relation to the anus in order to prevent inadvertent rectal discharge and staining of clothes.

U.S. Pat. No. 3,570,489 issued March 16, 1971 to Brown discloses a device for insertion into the anus to block undesirable rectal discharge. Brown discloses a device for insertion into to the rectum that is connected to fibrous material for absorbing the rectal discharge. In general, rectal insertion devices have not proven satisfactory in all cases as they require the patient to have a properly developed and functional anal sphincter muscles to secure the device in place. These devices can be insecure when the patient is active and mobile, and can be irritating in the anal canal by reason of mechanical irritation of the device against the tissue.

U.S. Pat. No. 4,484,919 issued November 27, 1984 to Sohn, et al. discloses a rectal dressing that includes an elongated absorbent pad that conforms to the natal cleft and a base that that is attached to a fastening agent that adheres to another part of the rectal area. A potential problem with this device is that mechanical forces resulting from body movements can cause the rectal dressing to shift within the natal cleft. This can cause discomfort, irritation, and chafing in the perianal area. Furthermore, any movement of the device away from the perianal area caused by natural body movements can result in rectal discharge that is not absorbed by the dressing.

U.S. Pat. No. 5,695,484 issued December 9, 1997 to Cox discloses an anal patch that is saddle shaped and has an adhesive coating on the surface of the patch to keep the patch in place. A potential problem with this device is that adhesive coatings can be irritating to the skin, especially in the context of treating hemorrhoids which are know to have skin that is more sensitive to irritants.

U.S. Pat. No. 4,702,237 issued October 27, 1987 to Gianopoulos, et al. teaches a hemorrhoid retainer designed to be adhered to the anal area to physically hold the hemorrhoids within the anal orifice. However, adhering such pads to the skin of the buttock can be painful and cause discomfort.

U.S. Pat. Appl. No. 2001/0003157 A1 published June 7, 2001 to Toth teaches a hemorrhoid relief and anal hygiene pad designed to apply pressure to the anal area. In particular, the pad applies pressure when placed between opposing buttock surfaces. The apparent large and bulky shape of the pad could make it very uncomfortable to use.

WO 00/02508 published January 20, 2000 to Gomez et al. discloses a sanitary towel intended to be used in the anal area. The towel has an arch or conical shape and a wedge-like extremity. However, it is not clear how the towel would be held in place, particularly for a relatively long period of time. These and other known pads and apparatuses have thus been less than fully effective for those desiring an effective sanitary and minimally invasive solution to the problem of hemorrhoids.

Accordingly, it is desirable to have a hemorrhoid treatment pad that can be worn for long periods relatively comfortably.

Further, it desirable to have a hemorrhoid treatment pad that can remain in place without the use of adhesives.

Finally, it is desirable to have a hemorrhoid treatment pad that can both ease discomfort, aid in improving conditions in the anal area, and absorb rectal discharges.

## SUMMARY OF THE INVENTION

A hemorrhoid treatment pad is disclosed, the hemorrhoid treatment pad having a substance on a body facing surface thereof, the substance comprising a skin care composition. The skin care composition can have from 0.001% to 0.1% by weight of hexamidine; from 0.001% to 10% by weight of zinc oxide; from 0.01% to 10% by weight of niacinamide; and a carrier.

## BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:

FIG. 1 is a plan view of a hemorrhoid treatment pad of the present invention.

FIG. 2 is a cross-sectional view of the pad of FIG. 1 through section 2-2.

FIG. 3 is a side elevation view of a hemorrhoid treatment pad of the present invention.

FIG. 4 is a perspective view of a hemorrhoid treatment pad of the present invention being handled.

FIG. 5 is a plan view schematic of the flushability test apparatus.

FIG. 6 is a cross-sectional representation of a portion of the flushability test apparatus.

## DETAILED DESCRIPTION OF THE INVENTION

The hemorrhoid treatment pad 20 shown in FIG. 1 has a longitudinal centerline L which runs along the "x" axis. The term "longitudinal", as used herein, refers to a line, axis or direction in the plane of the hemorrhoid treatment pad 20 that is generally aligned with (e.g., approximately parallel to) a vertical plane which bisects a standing wearer into left and right body halves when the hemorrhoid treatment pad 20 is worn. The terms "transverse," "lateral," or "y direction" as used herein, are interchangeable, and refer to a line, axis, or direction that is generally perpendicular to the longitudinal axis. The lateral direction is shown in FIG. 1 as the "y" direction. The hemorrhoid treatment pad 20 shown in FIG. 1 also has a transverse centerline T. The "z" direction, shown in FIG. 2, is a direction parallel to the vertical plane described above. The term "upper" refers to an orientation in the z-direction toward the wearer's head. "Lower" or "downwardly" is toward the wearer's feet.

The hemorrhoid treatment pad 20 as shown in FIGS. 1-3 has a body-facing (or "body-contacting") side 20A sometimes referred to as a top surface, and an opposed underside 20B. The hemorrhoid treatment pad comprises a pad-like main body portion (or "central absorbent portion") 22 and an optional placement and removal tab 52 which is joined to the underside 20B of the main body portion 22 to provide the overall hemorrhoid treatment pad with a "T"-shaped cross-sectional configuration. The main body portion 22 can be in any suitable configuration. Non-limiting examples of shapes for the main body portion 22 when viewed from the top as in FIG. 1 include ovoid, elliptical, trapezoidal, rectangular, triangular, diamond-shaped, or any combination of the above. As shown in FIG. 1, the preferred plan view shape for the main body portion 22 and the overall hemorrhoid treatment pad 20 is generally ovoid or elliptical. The plan view shape of the main body portion 22 tapers from the transverse centerline T towards its front and rear ends. The main body portion 22, in this embodiment, is relatively flat in its side profile, but may taper slightly from front to rear as shown in FIG. 3.

As shown in FIGS. 1-4, the hemorrhoid treatment pad preferably comprises a liquid pervious topsheet 42, a liquid impervious backsheet 38 joined to the topsheet 42, and an absorbent core 44 positioned between the topsheet 42 and the backsheet 38. In some embodiments it is not necessary that the topsheet be joined to the backsheet directly. The hemorrhoid treatment pad 20 is preferably of a size and shape that allows at least the majority of the pad 20 to fit comfortably within the wearer's natal cleft and to cover the wearer's anal orifice. The hemorrhoid treatment pad 20 at least partially blocks, and more preferably completely blocks and intercepts blood from hemorrhoids, rectal discharge, and/or other bodily exudates from around the wearer's anal orifice. The hemorrhoid treatment pad 20 can also provide soft, non-abrasive pressure to the wearers anus and natal cleft to relieve the pain associated with hemorrhoids.

The size of the hemorrhoid treatment pad 20 is important to its comfort and effectiveness. The length of the hemorrhoid treatment pad 20 is measured along the longitudinal centerline L in the longitudinal direction (or "x"-direction). The hemorrhoid treatment pad 20 preferably has a length $L_1$ which is greater than about 40 mm and less than about

130 mm. More preferably, the length $L_1$ is between about 60 mm and to about 100 mm. The width of the hemorrhoid treatment pad 20 is measured along the transverse centerline T in the transverse direction (or "y"-direction). The hemorrhoid treatment pad 20 preferably has a greatest width $W_1$ which is between about 25 mm and about 60 mm. The thickness (or caliper) is the "z"-direction dimension of the main body portion of the pad 20. Caliper measurements given herein were measured using an AMES gauge with a 0.25 psi (1.7 kPa) (gauge) load and a 0.96 inch (2.44 cm) diameter foot. Those skilled in the art will recognize that if a 0.96 inch (2.44 cm) diameter foot is not appropriate for a particular sample size, the foot size may be varied while the load on the gauge is accordingly varied to maintain a confining pressure of 0.25 psi (1.7 kPa) (gauge). The caliper $T_1$ of the main body portion of the hemorrhoid treatment pad 20 is preferably less than the width $W_1$ and the length $L_1$ of the pad 20. Preferably, the caliper $T_1$ of the main body portion of the hemorrhoid treatment pad 20 is less than or equal to about 8 mm, more preferably the caliper $T_1$ is less than or equal to about 6 mm, and even more preferably the caliper is less than or equal to about 4 mm.

The main body portion 22 of the hemorrhoid treatment pad 20 is preferably flexible enough to fold easily about the longitudinal axis, but has enough flexural rigidity to be held in place. This will increase the wearing comfort of the hemorrhoid treatment pad 20 and increase the likelihood of successful treatment and management of hemorrhoids. Preferably, the hemorrhoid treatment pad 20 has sufficient flexibility and flexural rigidity to adapt to any shape of the natal cleft. In any event, the flexure resistance should be sufficient to provide slight outward pressure on the wearer's buttock surfaces when positioned for use in the natal cleft.

The hemorrhoid treatment pad 20 preferably has sufficient absorbency to absorb and retain any exudates, e.g., blood or rectal leakage, discharged from the wearer's body. While actual capacity requirements can be negligible, it is desirable that the hemorrhoid treatment pad 20 have some absorbent capacity. The absorbent capacity is dependent at least partially upon the physical volume of the hemorrhoid treatment pad 20.

The absorbent core 44 of the hemorrhoid treatment pad 20 of the present invention can have a liquid capacity sufficient to absorb moderate amounts of rectal discharge. Preferably, the absorbent core 44 has a capacity to absorb at least 0.2 gram of rectal discharge, more preferably of at least 1.0 gram, and most preferably of at least 5.0 grams. The absorbent core 44 can have a total capacity of at least 10.0 grams.

The absorbent hemorrhoid treatment pad 20 preferably has a capacity of at least about 1 g of 0.9% by weight saline solution, and may have a capacity of up to about 30 g by using absorbent gels or foams that expand when wet. Preferably, capacities typically range from 2 to 10 grams, for saline, preferably from 0.1g to 5g, more preferably from 0.1g to 3g. Those skilled in the art will recognize that the capacity for absorption of rectal discharges such as blood will typically be smaller than the capacities given above for absorption of saline. A method for measuring absorbent capacity is by way of the Absorbent Capacity Test Method is disclosed in the Test Methods section, below.

The hemorrhoid treatment pad 20 as shown in its fully assembled configuration, preferably comprises at least one axis of preferred bending A. The axis of preferred bending A is preferably located generally along the longitudinal centerline L of the hemorrhoid treatment pad 20. The axis of preferred bending A is a line or axis along which the hemorrhoid treatment pad 20 will tend to bend or fold when subjected to compressive forces F directed inwardly in the transverse direction at the sides 32 of the pad 20. The axis of preferred bending A may result naturally from the product configuration, or the pad 20 may be imparted with a weakened axis or region in any or all of the topsheet 42, backsheet 38 and core 44 to create the axis of preferred bending A. Such a weakened axis may be created by any variety of known techniques such as scoring, pre-folding, slitting, or the like. The hemorrhoid treatment pad 20 may comprise a region of preferred bending made up of a plurality of axes of preferred bending. Any number of such axes may comprise such a region.

The hemorrhoid treatment pad 20 is folded along the axis of preferred bending A, as shown in FIG. 4, prior to insertion within the wearer's natal cleft. Once inserted, the pad 20 will preferably tend to unfold slightly keeping the topsheet 42 of the pad 20 in contact with the inner walls of the wearer's opposed buttocks surfaces. The pad 20 may be resiliently biased slightly along the axis of preferred bending A to increase the tendency of the pad 20 to unfold. This allows the folded pad 20 to act as a "spring" and, consequently, to increase the tendency of the topsheet 42 of the pad to remain in contact with the inner surfaces of the opposed buttocks surfaces when the hemorrhoid treatment pad 20 is in place. A pad 20 constructed according to the preferred embodiment described above, however, does not necessarily require any additional structural features to provide the ability to maintain such contact.

The hemorrhoid treatment pad 20 shown in FIGS. 1-3 (i.e. one in which the pad is tapered at the ends) allows the pad to easily and comfortably fit the wearer's natal cleft. A pad 20 with such a tapered shape, when folded along an axis of preferred bending A (as in FIG. 4) will have a profile in which highest point along the axis of bending A (as measured in the "z"-direction) is in the vicinity of the center of the pad 20 rather than at the ends.

As discussed more fully below with respect to a preferred lotion, the hemorrhoid treatment pad 20 preferably has a substance 46 deposited on the topsheet 42 prior to use, the substance 46 being capable of providing a certain amount of adhesion to hold the hemorrhoid treatment pad 20 in place during use. The substance 46 for holding the hemorrhoid treatment pad 20 in place preferably has certain additional characteristics. It should allow the hemorrhoid treatment pad 20 to be easily placed in the proper position without discomfort, and worn without irritation. It should also

preferably be biodegradable so that it is suitable for disposal in a toilet. The presence of the substance 46 should also not interfere with the flushability of the hemorrhoid treatment pad 20, if the hemorrhoid treatment pad 20 is of a flushable design. Preferred substances for holding the hemorrhoid treatment pad 20 in place are those which provide resistance to detachment under shear forces (such as those acting when the wearer walks), but can be comfortably removed using peeling forces.

The substance 46 for holding the hemorrhoid treatment pad 20 in place can include materials which are typically identified as adhesives, as well as materials which are not generally considered adhesives (that is, non-adhesive substances). Suitable adhesives include pressure sensitive adhesives and tacky non-pressure sensitive adhesive substances. Suitable pressure sensitive adhesives include silicone-based pressure sensitive adhesives such as polysiloxane, modified polysiloxanes, hydrocolloid-based adhesives, starch-based adhesives, moisture-activated adhesives, and any of known "body" adhesives.

Optionally, the hemorrhoid treatment pad 20 is provided with a non-adhesive substance 46 on its body-contacting surface to hold the hemorrhoid treatment pad 20 in place. The non-adhesive substance 46 can be of a type that has a "tack" (that is, stickiness), or it can be of a type that does not have a "tack". Suitable non-adhesive substances include waxes (such as microcrystalline waxes, paraffinic waxes, silicone waxes, polythylene waxes), fatty alcohols, high molecular weight alcohols, fatty acids, petroleum jelly, sealing ointments, non-ionic surfactants such as ethoxylated alcohols, ethoxylated long chain alcohols, and ethoxylated fatty acids, alkoxylated amide, alkoxylated amines, alkyl amido alkyl amines, alkyl substituted amino acids, moisture-activated substances, and combinations thereof. Another suitable non-adhesive substance 46 is the fat substitute OLEAN manufactured by the Procter & Gamble Company of Cincinnati, Ohio under U.S. Patent 5,085,884 issued February 4, 1992 and U.S. Patent 5,422,131 issued June 6, 1995, both to Young, et al. and U.S. Patent 5,422,131 issued to Elsen, et al. Without wishing to be bound by any particular theory, it is believed that such materials may hold an object in place due to high viscosity or surface tension.

The substance 46 for holding the hemorrhoid treatment pad 20 in place can be combined with other substances before it is applied to the hemorrhoid treatment pad. Such other substances can serve as a component of the substance 46 for holding the hemorrhoid treatment pad 20 in place, or as a carrier for the substance 46 for holding the hemorrhoid treatment pad 20 in place. Non-limiting examples of substances that can serve in either of these manners are lotions, emollients, and mineral oil. For example, the substance 46 for holding the hemorrhoid treatment pad in place can be a polyethylene glycol that is mixed in a lotion formula that provides lubricity during the insertion process and develops tack when contacted by moisture. In another example, an emollient can be used as a carrier for PEG's which are in particulate form. In still another example, the PEG's can be in liquid form, and can serve as a carrier for other materials.

The substance 46 described above can be applied to the body-contacting surface of the hemorrhoid treatment pad 20 in an intermittent pattern, a continuous pattern, or in a pattern that has both intermittent and non-uniform portions. By "intermittent" it is meant that the amount, location, pattern of distribution, etc. of the lotion composition can vary over the topsheet surface. For example, some portions of the treated surface of the topsheet can have greater or lesser amounts of lotion composition, including portions of the surface that do not have any lotion composition on it. Applying the substances in an intermittent pattern, such as in a longitudinally-oriented stripe (or stripes), may be useful if it is desired to minimize interference of the substances with acquisition of liquids into the hemorrhoid treatment pad 20 since liquids can be transported into the absorbent core between the intermittent zones of the substance. Applying the substances in a continuous pattern may be useful if it is desired to use the contact that the substance 46 makes to the wearer's body to create a barrier to the flow of exudates over the body-contacting surface of the hemorrhoid treatment pad. However, the application of the substances in a continuous pattern should not form an impermeable barrier which prevents rectal discharges, blood, or feces from being absorbed by the hemorrhoid treatment pad 20.

Where the substance 46 is applied intermittently, any pattern may be utilized, including, for example, application of small droplets (obtained via, e.g., spraying) discrete dots (obtained via, e.g., gravure printing), alternating stripes that run in the longitudinal or lateral direction of the article, etc. By alternating stripes is meant regions in which the lotion is applied as stripes separated by regions which have no lotion applied. The substance 46 can be applied directly to the hemorrhoid treatment pad 20, or it may be applied to another material or component which is then adhered to the desired portion of the hemorrhoid treatment pad 20. The substance 46 can cover any of the following percentages of the surface area of the body-contacting surface of the main body portion 22, the central absorbent portion, the flexible extensions, or the entire body-contacting surface of the hemorrhoid treatment pad In those embodiments where the body-contacting surface of the main body portion 22, comprises discrete, untreated regions, the percent open area of the region of the body-contacting surface can vary widely. (As referred to herein, the "percent open area" of the body-contacting surface is determined by (i) measuring the surface area of the body-contacting surface, (ii) measuring the total surface area of the untreated region(s) of the body-contacting surface and (iii) dividing the measurement in (ii) by the measurement in (i). As used herein, "untreated" means a region of the body-contacting surface having less than 0.05 g/m2 of substance 46. In this regard, the percent open area may be from 1% to 99%, from 5% to 95%, from 10% to 90%, from 15% to 85%, from 20% to 80%, from 25% to 75%, from 30% to 70%, or from 35% to 65%. The percent open area required to achieve the desired substance 46 effect and the desired fluid handling properties of the topsheet will be dictated largely by the

characteristics of the substance 46 (in particular the lotion's composition and its relative hydrophobicity / hydrophilicy properties).

For certain lotion compositions (e.g., those that are hydrophobic), the untreated regions of topsheet 42 further facilitate rectal discharge passage into the absorbent core. Surprisingly, while the topsheet is treated intermittently (e.g., the topsheet has microscopic or macroscopic regions where no lotion is applied), during wear of the article, lotion is transferred to the wearer even in regions of the skin corresponding to non-lotion regions of the topsheet. The amount and uniformity of lotion transferred to the skin is believed to depend on several factors, including, for example, contact of the wearer's skin to the topsheet, friction created during wear time between the wearer's skin and the treated topsheet, warmth generated from wearer to enhance the transfer of the lotion, the lotion properties, lotion composition, and the like.

In one preferred embodiment, the topsheet comprises stripes of lotion that run in the article's longitudinal direction. In such embodiments, each lotion stripe can typically have a width of from 1.0 mm to 24.0 mm, more typically from 1.0 mm to 12 mm, and the width of the stripes containing no lotion can typically be from 1.0 mm to 24 mm, more typically from 1.0 mm to 12 mm.

The substance 46 can be placed on any suitable portion of the hemorrhoid treatment pad 20. The substance 46 can be placed on the entire body-contacting surface of the hemorrhoid treatment pad 20, or on a portion thereof. For example, the substance 46 can be placed on all or a portion of the body-contacting surface of the main body portion 22. If the hemorrhoid treatment pad is of a type that comprises a central absorbent portion and flexible extensions extending therefrom, the substance 46 can be placed on the central absorbent portion, the flexible extensions, or both the central absorbent portion and the flexible extensions. The substance 46 can, thus, be placed on a central region of the hemorrhoid treatment pad 20, but not on the peripheral portions of the hemorrhoid treatment pad. More preferably, however, the substance 46 may be placed on the peripheral portions of the body-contacting surface of the hemorrhoid treatment pad, but not in the central region. Locating the substance 46 in the latter manner may be advantageous if it is desired to minimize any tendency for the substance 46 to interfere with sensitive tissues around the anal orifice. The substance 46 can also be used to create a seal to prevent the flow of exudates toward the ends (and/or sides) of the pad. In a preferred embodiment, the substance 46 can be applied along an edge of the body contacting surface, or example, in a stripe extending from an edge approximately 5 mm.

The substance 46 can be applied to the hemorrhoid treatment pad 20 in any suitable quantity. For these purposes, the quantity of the substance 46 applied to the hemorrhoid treatment pad 20 can be expressed as a percentage of the total product weight including the pad and the weight of the substance 46. Preferably, the substance 46 constitutes less than or equal to about 20%, more preferably less than or equal to about 10%, and most preferably less than or equal to about 5% of the total product weight, so as not to excessively contribute to the overall weight of the hemorrhoid treatment pad. This permits more of the total product weight to be dedicated to providing absorbent capacity.

Alternatively, the quantity of the substance 46 applied to the hemorrhoid treatment pad 20 can be expressed as an amount applied to the body contacting surface of the topsheet. Preferably, the substance 46 can be applied from 0.0155 g/$m^2$ (0.01 mg/$in^2$) to 310 g/$m^2$ (200 mg/$in^2$) preferably from 0.155 g/$m^2$ (0.1 mg/$in^2$) to 155 g/$m^2$ (100 mg/$in^2$), more preferably 0.5 g/ $m^2$ (0.32 mg/ $in^2$) to 93 g/$m^2$ (60 mg/$in^2$), to the absorbent article. Of course, for articles having relatively high percent open areas in the body contacting surface, greater add-on levels may be obtainable without adversely affecting fluid handling by the topsheet.

There are many possible specific embodiments of hemorrhoid treatment pads with various substances thereon for assisting the hemorrhoid treatment pad in staying in place in the desired position in the natal cleft. The hemorrhoid treatment pad can have one or more of the substances described herein applied thereto in any of the patterns of application described herein. A preferred lotion substance 46 is described below.

In addition to the various embodiments of the substances for holding the hemorrhoid treatment pad 20 in place which are described herein, the hemorrhoid treatment pad can be provided with other optional features. For example, the hemorrhoid treatment pad of the present invention can provide a benefit to the user in controlling odors associated with body exudates. Additional odor controlling agents may be added to the absorbent core for further reductions in odors. Such odor controlling agents include, but are not limited to activated charcoals, zeolites, silica, polyacrylic acids (superabsorbents), certain quaternary compounds, triethyl citrate, cyclodextrin, or any combinations thereof. Particularly preferred cyclodextrin compounds are described in U.S. Patent 5,429,628 issued to Trihn, et al. and U.S. Patent 5,780,020 issued to Peterson, et al. In addition, deodorants can be added to further mask these odors.

Further, over-the-counter anal drug actives can be added to the body contacting surface for one or more of the following purposes: cleansing, providing soothing and refreshing effects, deodorizing, relieving minor irritation, reducing the number of pathogenic microorganisms, or producing an astringent effect. Such over-the-counter anal drug actives include: calcium propionate, dioctyl sodium sulfosuccinate, nonoxynol 9, octoxynol 9, potassium sorbate, povidone-Iodine (PVP-Iodine), sodium lauryl sulfate, corticosteroids, pramoxine-HCl, bacitracin, neomycin, polymyxin B sulfate, tetracyclines, triclosan, and sodium propionate.

The individual components which may be suitable for the various embodiments of the hemorrhoid treatment pad 20 are now disclosed in greater detail with reference to FIGS. 1-4.

**TOPSHEET**

The top surface of pad 20 can be a topsheet 42 which is liquid pervious, permitting liquids (e.g., blood or rectal leakage) to readily penetrate through its thickness. The topsheet 42 should be compliant, soft feeling, and non-irritating to the wearer's skin.

A suitable topsheet 42 may be manufactured from a range of materials such as nonwoven materials and polymeric materials such as hydroformed thermoplastic films. Suitable nonwoven materials can be comprised of natural fibers (e.g., hydrophobically-treated wood, rayon, or cotton fibers), synthetic fibers (e.g., polymeric fibers such as polyester, rayon, polypropylene, or polyethylene fibers) or from a combination of natural and synthetic fibers. If the topsheet comprises a nonwoven material, it can be made by any suitable process. For example, it can be carded, spunbonded, meltblown, needlepunched, etc.

The topsheet materials described above (nonwovens, films, etc.) can be made from biodegradable materials, or non-biodegradable materials. As used herein, the term "biodegradable materials" refers to materials having greater than or equal to 70% biodegradation (percentage of theoretical carbon dioxide evolution) after 28 days when measured according to the Sturm Test which has been designated Method 301B by the Organization for Economic Cooperation and Development, 2 rue Andre Pascal, 75775 Paris Cedex 16, France. Preferably, the materials comprising the hemorrhoid treatment pad of the present invention have a biodegradation of greater than 80% and, more preferably, biodegradation is greater than or equal to about 90%.

The topsheet 42 can be provided with the improved properties described herein so that it will be more comfortable for contacting the wearer's sensitive anal tissues, including protruding hemorrhoids. The topsheet preferably comprises a non-absorbent, moderately hydrophilic to substantially hydrophobic material. In preferred embodiments, the topsheet is made as hydrophobic as possible to reduce the tendency for it to adhere to the hydrous body tissues. Hydrophobicity a material can be measured by measuring the critical surface tension of the material. The topsheet 42 preferably has a critical surface tension of less than or equal to about 45 dynes/cm, more preferably less than or equal to about 40 dynes/cm. The critical surface tension is measured according to the ASTM method D 2578-94, for measuring the wetting tension of polymer films. This method will work with nonwoven materials as well. Examples of critical surface tension for materials useful as topsheets in the present invention include COROLIND, available from BBA Nonwovens, Peine, Germany, 29-30 dynes/cm; and BIONELLE 3001 obtained from Showa Hugh Polymer Co. of Tokyo, Japan, 40 dynes/cm.

The topsheet 42 should preferably be compressible and resilient so that it is comfortable when placed adjacent to the wearer's anal orifice. The compressibility is preferably measured under loads typically encountered when the user wears a product having the topsheet thereon. The topsheet can be a nonwoven layer having easily deformable ridges and valleys, such as ridges formed by processes commonly known as "ring rolling", or by the process described in U.S. Pat. No. 5,518,801 issued to Chappell. The topsheet can also comprise a film layer, such as a three-dimensional, apertured, formed film. Such films are known for sanitary napkins, for example, and can be made as shown in U.S. Pat. Nos. 4,609,518, and 4,629,643, issued to Curro et al.

The topsheet preferably undergoes a caliper change of greater than or equal to about 30% under a pressure of 1,000 Pa after being subjected to a pressure of 250 Pa. The topsheet 42 preferably undergoes an absolute caliper change of greater than or equal to about 0.15 mm under a pressure of 1,000 Pa after being subjected to a pressure of 250 Pa. The topsheet compressibility can be measured according to the Topsheet Compressibility (Thickness Change) Test Method below.

The topsheet 42 is preferably also extensible so that it can be capable of extending with the movements of the wearer's body, and specifically with the movements of the wearer's buttocks surfaces. Anatomically speaking, the anal orifice resides in a relatively low motion zone of the body. However, normal body motions associated with walking, and even sitting, can cause relative motion between the opposing surfaces of the buttocks in the anal cleft. Therefore, to reduce abrasion and the associated discomfort it produces, the topsheet is preferably extensible in at least one direction in an amount greater than or equal to 30% under a force of 50 grams. The topsheet 42 may be extensible in one direction, in two directions, in multiple directions, or in all directions (i.e., it may be omni-directionally extensible). In some embodiments the topsheet 42 is not only extensible, but is also elastically extensible. By "elastically extensible" is meant that upon extension and after release of the extension force, the material returns to at least 10% of its original dimension. Having an elastic extensibility of at least about 10%, more preferably 25% or even 100% helps prevent wrinkling of the product during use.

In other embodiments, a topsheet 42 may be provided which is comfortable when one of the qualities quantified herein is lower than set forth herein, if one of the other qualities of the topsheet quantified herein is increased. For instance, in one non-limiting example, the topsheet 42 may have a different combination of compressibility and extensibility, if desired. For example, a suitable topsheet may have an extensibility in at least one direction of greater than or equal to about 20% under a force of 50 grams, and the topsheet may undergo a caliper change of greater than or equal to about 40% when tested according to the Topsheet Compressibility (Thickness Change) Test, described in the Test Methods section, below.

The topsheet 42 is preferably joined to the other components of the absorbent article such that the topsheet maintains it ability to stretch in response to the motions of the wearer's body. The inner surface of topsheet 42 may be secured in contacting relation with an underlying absorbent layer. The topsheet 42 may be kept in a contacting relationship with an underlying layer by bonding the topsheet 42 to the underlying layer. However, it is not absolutely necessary to bond the face of the topsheet 42 to the face of the underlying layer. The topsheet 42 can be maintained in contact with an underlying absorbent component by entangling the fibers of the underlying layer with the topsheet, by fusing the topsheet 42 to an underlying absorbent layer by a plurality of discrete individual fusion bonds, or by any other means known in the art. The topsheet can also be maintained in contact with the underlying absorbent material due to the application of the pressure of the body against the body-contacting surface of the hemorrhoid treatment pad.

An example of a topsheet suitable for topsheets of the present invention is a hi-loft web manufactured in either an airlaid process or a carded process. For example a suitable web can be manufactured by airlaying a blend of staple fibers having a length of approximately 8 mm, the blend comprising about 10-25% bi-component binding fibers and thermally bonding the airlaid web without calendaring. For example a fiber blend of 40% 8 mm long PET staple fibers available as Celanese #295 from Celanese AG (Summit, NJ, USA)), 40% 8 mm long, 1.25 denier Lyocel available from Tencel Inc. Mobile, A1 and 20% 8 mm long, 1.2 denier bi-component fiber (8 mm, 1.2 denier, C1.7 dtex, FiberVisions AL Adhesion United States) can be airlaid and thermally bonded without calendaring to a basis weight of 20 grams per square meter (gsm). Ideally the constituent 8 mm fibers are of a relatively low denier (e.g., <2 denier) to improve the webs softness. Alternatively, a calendaring process (using longer fibers of the same fibers disclosed above) can be used to lay down such a web followed by a thermal bonding step using through air bonding to effectively point bond individual fibers within the web.

## ABSORBENT CORE

Unlike other absorbent articles, such as sanitary napkins, interlabial products, and the like, the absorbent core of the present invention need not have a relatively high liquid absorbing capacity. In some cases, the hemorrhoid treatment pad of the present invention can be used without the need for any absorbent capacity. That is, the benefits of the hemorrhoid treatment pad of the present invention can be simply providing pressure-induced comfort to the anal area, without the need to absorb blood from external hemorrhoids or rectal discharges. However, the product preferably has some absorbent capacity, and therefore, the following description of the absorbent core 44 is for a preferred embodiment.

The absorbent core 44, which is best seen in FIG. 2, is positioned between the topsheet 42 and the backsheet 38. The absorbent core 44 provides softness, as well as the means for absorbing exudates such as rectal discharges, blood, and other body fluids. The absorbent core 44 preferably is generally compressible, conformable, and non-irritating to the user's skin. The absorbent core 44 can be of any suitable size and shape. Preferably, the absorbent core 44 has the same general shape as the overall hemorrhoid treatment pad 20.

The absorbent core 44 may comprise any suitable material that is capable of absorbing and/or retaining liquids (e.g. blood). The absorbent core 44 can be manufactured from a wide variety of liquid-absorbent materials commonly used in absorbent articles such as comminuted wood pulp which is generally referred to as airfelt. Examples of other suitable absorbent materials include cotton fibers or cotton lintels, creped cellulose wadding; meltblown polymers including conform; chemically stiffened, modified or crosslinked cellulosic fibers; synthetic fibers such as crimped polyester fibers; peat moss; tissue including tissue wraps and tissue laminates; absorbent foams; absorbent sponges; superabsorbent polymers (in fibrous and particulate form); absorbent gelling materials; or any equivalent material or combinations of materials, or mixtures of these. Preferred absorbent materials comprise folded tissues, cotton batts, woven materials, nonwoven webs, rayon including needle punched rayon, and thin layers of foam. The absorbent core 44 may comprise a single material or a combination of materials.

One preferred material for the absorbent core 44 is a batt of rayon or a rayon/cotton blend. In one embodiment, the absorbent core 44 is a batt of fibers which comprises a 50%/50% blend of baled bleached cotton fibers and baled rayon fibers. A tri-lobal rayon known as GALAXY rayon available from Acordis of Mobile, Alabama USA has been found to work well for the material comprising the absorbent core 44.

The absorbent core 44 can comprise fibrous superabsorbent material in any suitable amount. Alternatively, instead of comprising fibrous superabsorbent material, the superabsorbent material can be in the form of particles, or any other form known in the art. In other embodiments, the absorbent core 44 can comprise superabsorbent material in a combination of different forms.

The absorbent core 44 can comprise any suitable structure that is capable of providing soft, gentle pressure to the natal cleft when the hemorrhoid treatment pad in use, as well as efficiently storing body discharges that are received from the topsheet 42, and any intermediate layers, such as an acquisition layer (not shown) between the topsheet 42 and the absorbent core 44. In one embodiment, the absorbent core 44 comprises a 300 $g/m^2$ airlaid layer comprised of superabsorbent fibers, airfelt, and bicomponent fibers. Although the superabsorbent fibers can be distributed in any suitable manner within the absorbent core, the superabsorbent fibers are preferably distributed substantially

uniformly throughout the absorbent core 44. The absorbent core 44 can comprise a high concentration (greater than or equal to 25% by weight) of a fibrous superabsorbent material (or absorbent gelling material), preferably a high gel strength fibrous superabsorbent material.

In one embodiment, the topsheet and an absorbent core are integrally formed by needle-punching a nonwoven layer through a fibrous web of fibrous absorbent gelling materials. Such a topsheet/core is disclosed in U.S. Pat. Applications 09/637,440 or 09/905,804. These applications published 21 February 2002 as WO 02/13750. Further, as disclosed in one or both of the two applications referenced above, the pad 20 can be die cut to shape, with or without further bonding about the die-cut periphery. In such an embodiment, each layer can be assembled and joined as desired, and the entire layered structure can be die-cut to form the finished pad 20.

## BACKSHEET

Because the primary purpose of the hemorrhoid treatment pad 20 of the present invention is not to absorb and retain large amounts of fluid, the backsheet 38 as disclosed herein can be optional. However, because the product will likely at times be required to absorb at least a small amount of a rectal discharge, including blood, a backsheet is preferably included. Additionally, it is foreseeable that the hemorrhoid treatment pad of the present invention may be used for light fecal incontinence. Therefore, preferably a backsheet is included to prevent any fecal matter from passing through the pad to the wearers skin or clothing.

The backsheet 38, which is best shown in FIGS. 2 and 3, prevents any exudates absorbed and contained in the absorbent core 44 from wetting articles and/or body parts which may contact the hemorrhoid treatment pad 20 such as pants, pajamas, and undergarments. The backsheet 38 should be flexible and impervious to liquids (e.g., blood, feces). As used herein, the term "flexible" refers to materials which are compliant and will readily conform to the general shape and contours of the human body. The backsheet 38 also provides protection for the wearer's fingers as the hemorrhoid treatment pad 20 is inserted, or, if necessary, as the pad is removed with the fingers.

The backsheet 38 may comprise a woven or nonwoven material, polymeric films such as thermoplastic films of polyethylene or polypropylene, composite materials such as a film-coated nonwoven material, or organic material such as a collagen film. Other suitable materials include dispersible materials such as polyvinyl alcohol, and biodegradable polymers that can be made into films and the like. Suitable biodegradable polymers include: BIONELLE 3001 obtained from Showa Hugh Polymer Co. of Tokyo, Japan; BAK 403 biodegradable polymer obtained from Bayer AG of Leverkusen, Germany; Matter Bi ZF03U-A obtained from Bicorp Co., distributor for Novamont S.P.A. of Rome, Italy; and, Biopol biodegradable polymer obtained from Monsanto. In one embodiment, the backsheet may be made from a polyethylene film having a thickness of from 0.012 mm (0.5 mil) to 0.051 mm (2.0 mils). An exemplary polyethylene film is manufactured by Clopay Corporation of Cincinnati, Ohio, under the designation P18-0401. Preferably, however, the backsheet 38 comprises a film having a similar thickness to this polyethylene film, and which is made of a biodegradable polymer such as the BIONELLE biodegradable polymer described above.

The backsheet 38 may also permit vapors to escape from the hemorrhoid treatment pad 20 (i.e., be breathable) while still preventing exudates from passing through the backsheet. A suitable breathable backsheet material is a laminate of an apertured film such as that described in U.S. Patent 3,929,135 issued to Thompson which is inverted so that the smaller openings of the tapered capillaries face the absorbent core 44 which is adhesively laminated to a microporous film such as that described in Exxon's U.S. Patent 4,777,073.

In preferred embodiments, the backsheet 38 is dispersible and/or dissolvable in water. Polyvinyl alcohol (including co-polymers of polyvinyl alcohol) has been found to be suitable as a material for a dissolvable backsheet 38. The polyvinyl alcohol may be coated on a tissue, a nonwoven material such as a biodegradable nonwoven material (e.g. rayon), or coated with a wax, such as paraffin, or other hydrophobic coating to reduce the rate at which it dissolves in water. This allows the backsheet 38 to maintain its integrity during use, while retaining the ability to dissolve in water during disposal of the hemorrhoid treatment pad 20.

In preferred embodiments, the backsheet 38 is dispersible and/or dissolvable in water. The term "dispersible", as applied herein to a hemorrhoid treatment pad or a component thereof, refers to an article or material which will disperse into at least two fragments in mildly agitated water. Such a pad will break into pieces in a conventional toilet and/or domestic plumbing system, and will ultimately be effectively processed though a sewage treatment system. The term "dissolvable", as applied herein to an hemorrhoid treatment pad or a component thereof, refers to an article or material which will at least partially dissolve and essentially assume liquid form or otherwise be indistinguishable to the naked eye from the liquid medium in which it is dissolved.

In other embodiments, the backsheet can be eliminated. In some embodiments, the backsheet can be eliminated if the underside of the absorbent core is coated, or otherwise treated, to make the underside resistant to the passage of liquids therethrough. Alternatively, when the hemorrhoid treatment pad 20 is folded along a longitudinal axis A (as shown in FIG. 4), especially if the pad is primarily contained deep within the natal cleft, the underside of the two halves of the hemorrhoid treatment pad 20 which would normally be provided with a backsheet, will contact each other (that

is, after the user's fingers are removed), and the main body portion of the pad 20 will assume an inverted V or U-shaped cross-sectional structure. In this case, the portion of the pad 20 which would normally be provided with a backsheet will not contact a wearer's garments, so the backsheet will not be necessary. The elimination of a backsheet improves the breathability of the pad.

The components of the hemorrhoid treatment pad 20 described above (topsheet 42, backsheet 38, if present, and absorbent core 44, if present) can be assembled in any suitable manner. In the embodiment shown in FIGS. 1-3, the components of the main body portion 22 are assembled in a "sandwich" configuration with the components sized so that the edges of the topsheet 42 and backsheet 38 extend outward beyond the edges of the absorbent core 44.

The components of the hemorrhoid treatment pad 20 can be joined together in any suitable manner. The term "joined," as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with the another element, i.e., one element is essentially part of the other element.

In the embodiment shown in FIGS. 1-3, the topsheet 42 and backsheet 38 are preferably at least partially peripherally joined using known techniques. As shown in FIGS. 1 and 2, the topsheet 42 is secured to backsheet 38 along an optional seam 60. The seam 60 is optional because the pad 20 can provide effective relief and treatment without the seem 60. Seam 60 is preferably liquid impervious. The seam 60 can be formed by any means commonly used in the art for this purpose such as by gluing, crimping, or heat-sealing. The seam 60 and the area of the hemorrhoid treatment pad 20 in the vicinity of the seam 60 should be soft, compressible, and conformable. If the seam 60 and surrounding area are too stiff or non-compressible, the wearer may experience discomfort when wearing the hemorrhoid treatment pad 20.

Instead of, or in addition to, the peripheral seam 60, the components of the hemorrhoid treatment pad 20 can be joined together at their faces. The faces of the components of the hemorrhoid treatment pad 20 can be joined together by adhesives, stitching, heat and/or pressure bonds, dynamic mechanical bonds, ultrasonic bonds, intermingling or entanglement of the fibers or other structural elements comprising the components of the hemorrhoid treatment pad 20, such as by meltblowing the fibers comprising one component onto another component, extruding one component onto another, or by any other means known in the art. The components of the hemorrhoid treatment pad 20 may be joined with water soluble adhesives in order to increase the tendency of the pad 20 to disperse into a plurality of fragments in mildly agitated water (such as in a toilet). It is, therefore, desirable that the material joining the components lose strength when exposed to an excess of water, such as when placed in a toilet. Water soluble or water dispersible adhesives, such as those based on carboxymethyl cellulose, polyvinyl alcohols, starches, and the like are well known in the art.

As previously discussed, the hemorrhoid treatment pad 20 is designed to be placed within the natal cleft of a wearer. As shown in FIG. 4, to use the hemorrhoid treatment pad 20, the wearer grasps an insertion aid, such as tab 52 of the pad 20. If the pad 20 is not provided with a tab 52, the wearer may hold the folded pad 20 at the sides 32 and begin insertion. As shown in FIG. 4, the pad 20 is then further inserted by pushing with a fmger or fingers in the recess 62 formed by the folded backsheet 38. Recess 62 covers the tips of the wearer's fingers during insertion. This feature provides for a hygienic insertion of the hemorrhoid treatment pad 20 of the present invention. The wearer may assume a squatting position during insertion to assist in spreading the buttocks surfaces.

Tab 52 can also be used as a removal aid, if necessary. In other embodiments strings, loops or the like can also be used as a removal aid. For example, an attached cotton string such as those used for tampon catamenial devices can be used, the string remaining substantially out of the natal cleft and being long enough for the user to grasp and pull to remove pad 20.

The tab 52, if used, may be made of a variety of materials and need not be absorbent. In one example, the tab 52 may be formed from a nonwoven material which is heat bonded to a tissue layer. A suitable nonwoven material is COROLIND available from BBA Nonwovens, Peine, Germany.. A suitable airlaid tissue is available from Merfin Hygenic Products, Ltd., of Delta, British Columbia, Canada, having a basis weight of about 61 gsm and having the designation grade number 176.

## OTHER ASPECTS OF THE HEMORRHOID TREATMENT PAD

Preferably, the hemorrhoid treatment pad 20 of the present invention is toilet-disposable. The term "toilet-disposable", as used herein, means that the hemorrhoid treatment pad is capable of being disposed of in a toilet. The hemorrhoid treatment pad is preferably at least flushable. In particularly preferred embodiments, the hemorrhoid treatment pad may also be provided with one or more of the following characteristics: dispersibility, settleability, disintegrateability, and biodegradability.

As used herein, the terms "flushable" and "flushability" refer to a product's ability to pass though typically commercially available household toilets and plumbing drainage systems without causing clogging or similar problems that can be directly associated with the physical structure of the product. It is recognized, however, that there can be

many differences between the various types of toilets available.

Preferably, the hemorrhoid treatment pad 20 of the present invention is dispersible and will disperse into at least two fragments within two hours of exposure to mildly agitated room temperature water as described in the Water Dispersion Test in the Test Methods section, below. More preferably, the hemorrhoid treatment pad 20 will be dispersed into at least two fragments within about 60 minutes or, even more preferably within about 30 minutes, and most preferably, within about 15 minutes. Preferably, the product will break into at least two fragments in which individual fragments are smaller than 38,7 $cm^2$ (6 $in^2$) more preferably smaller than 25,8 $cm^2$ (4 $in^2$), most preferably smaller than 12,9 $cm^2$ 2 $in^2$. In particularly preferred embodiments of the present invention, each of the components of the hemorrhoid treatment pad 20 will disperse into a plurality of fragments when immersed in mildly agitated water. Alternatively, the components of the hemorrhoid treatment pad 20 may separate from each other without themselves breaking into a plurality of fragments (e.g. the topsheet 42, backsheet 38, and core 44 may break apart from each other while each otherwise remaining intact).

"Settleability" refers to the tendency of an hemorrhoid treatment pad, such as hemorrhoid treatment pad 20 to eventually settle to the bottom of a septic tank or other sewage treatment system rather than to float on the surface of such tanks or sewage being processed.

Disintegrateability and biodegradability can be measured in accordance with the 28 Day Sludge Test which is in the Test Methods section below. Preferably, the hemorrhoid treatment pad 20 comprises biodegradable materials. While biodegradable materials are preferred for the hemorrhoid treatment pad 20, it is not necessary that each and every material used be biodegradable. For example, the hemorrhoid treatment pad 20 may comprise superabsorbent particles which do not biodegrade, and this will not affect the ability of the overall hemorrhoid treatment pad 20 to remain toilet-disposable and to be effectively processed in a sewage treatment system. On an overall weight basis, the hemorrhoid treatment pad 20 is preferably at least about 70% biodegradable, more preferably at least about 80% biodegradable, more preferably still at least about 90% biodegradable, and most preferably, at least about 95% biodegradable.

<u>Preferred Lotion</u>

The substance 46 comprises a skin care composition comprising a combination of the skin treatment agents hexamidine, zinc oxide, and niacinamide which are highly effective in the prevention and treatment of erythema, malodor, and bacterial skin disorders, especially when these skin care compositions are administered to the skin from application on absorbent articles.

The term "skin treatment agent" as used herein refers to materials that when applied topically and internally to the skin are capable of preventing, reducing, and/or eliminating any occurrence of skin disorders, particularly skin disorders associated with erythema, malodor, and bacterial infections.

The term "skin disorders" as used herein refers to symptoms associated with irritating, acute, or chronic skin abnormalities. Examples of such symptoms include, but are not limited to, itching, inflammation, rash, burning, stinging, redness, swelling, sensitivity, sensation of heat, flaking/scaling, malodor, and the like.

The term "ambient conditions" as used herein refers to surrounding conditions at about one atmosphere of pressure, at about 50% relative humidity, at about 25°C.

The skin care compositions can comprise, consist of, or consist essentially of the elements and limitations of the invention described herein, as well as any of the additional or optional ingredients, components, or limitations described herein.

All percentages, parts and ratios are by weight of the total composition, unless otherwise specified. All such weights as they pertain to listed ingredients are based on the specific ingredient level and, therefore, do not include carriers or by-products that may be included in commercially available materials, unless otherwise specified.

**<u>Skin Treatment Agents</u>**

The skin care compositions comprise skin treatment agents that are capable of reducing and eliminating the occurrence of skin disorders that can result from contact between the skin and moisture-laden air, skin disorders resulting from prolonged moist human tissue that can occur from the skin being exposed to moisture or other body exudates, or skin disorders that are generated from contact between the skin and microbial or bacterial agents. The skin treatment agents include hexamidine, zinc oxide, and niacinamide, preferably a select combination of these skin treatment agents wherein the combination provides improved reduction of skin disorders when the combination is administered in relatively low individual and combined concentrations.

The combination of skin treatment agents such as hexamidine, zinc oxide, and niacinamide have been found to be equal or more effective in the treatment of skin disorders as compared to skin formulations comprising these compounds individually at effective concentrations greater than an effective combined concentration of these compounds. Typically, skin treatment agents such as hexamidine, zinc oxide, and niacinamide are included in skin care compositions

at individual concentrations higher than those employed in the skin care compositions of the present invention. The skin care compositions include hexamidine at a concentration of 0.1% or less by weight, zinc oxide at a concentration of less than 30% by weight, and niacinamide at a concentration of about 3% or less by weight to achieve equal or superior benefits in the prevention and/or treatment of skin disorders as compared to known skin care compositions that generally comprise these skin treatment agents at about 1% or greater by weight of hexamidine, about 40% or greater by weight of zinc oxide, and about 4% or greater by weight of naicinamide.

Since the skin care compositions have been found to be effective in the treatment of skin disorders with the administering of such low levels of the hexamidine, zinc oxide, and niacinamide skin treatment agents, the total effective concentration of these skin treatment agents in the compositions are also lower than most typical combined concentrations of these ingredients. However, the total concentration of the skin treatment agents included in the skin care compositions herein are not limited to total concentrations of hexamidine, zinc oxide, and niacinamide. The skin care compositions of the present invention have also been found effective in the treatment of skin disorders when the compositions only contain low total concentrations of a combination of hexamidine and zinc oxide skin treatment agents, or low total concentrations of a combination of hexamidine and niacinamide skin treatment agents. Therefore, the total concentration of the hexamidine, zinc oxide, and niacinamide skin treatment agents in any combination ranges from 0.002% to 34%, preferably from 0.01% to 25%, more preferably from 0.1% to 2% by weight of the skin care composition. The hexamidine, zinc oxide, and niacinamide skin treatment agents are described in detail hereinbelow.

Although the skin care compositions preferably comprise a combination of hexamidine, zinc oxide, and niacinamide skin treatment agents, the skin care compositions can also comprise any other known or otherwise effective skin treatment actives. Nonlimiting examples of other suitable skin treatment actives include allantoin; aluminum hydroxide gel; calamine; cysteine hydrochloride; racemic methionine; sodium bicarbonate; Vitamin C and derivatives thereof; protease inhibitors including serine proteases, metalloproteases, cysteine proteases, aspartyl proteases, peptidases, and phenylsulfonyl fluorides; lipases; esterases including diesterases; ureases; amylases; elastases; nucleases; guanidinobenzoic acid and its salts and derivatives; herbal extracts including chamomile; and mixtures thereof. Guanidinobenzoic acid and its salts and derivatives are more fully described in U.S. Patent 5,376,655, issued to Imaki et al. on December 27, 1994. These other suitable skin treatment actives are typically included at concentrations ranging from 0.001% to 10% by weight of the skin care composition.

<u>Hexamidine</u>

The skin care compositions of the present invention comprise hexamidine skin treatment agent at concentrations ranging from 0.001% to 0.1%, preferably from 0.005% to 0.1%, more preferably from 0.01% to 0.1% by weight of the composition. The hexamidine, along with other skin treatment agents such as zinc oxide and niacinamide, provide highly effective skin treatment benefits even though these skin treatment agents are used at individual and combined concentrations that are lower than those typically used in the treatment of skin disorders.

The hexamidine skin treatment agent suitable for use herein include those aromatic diamines which generally conform to the following formula:

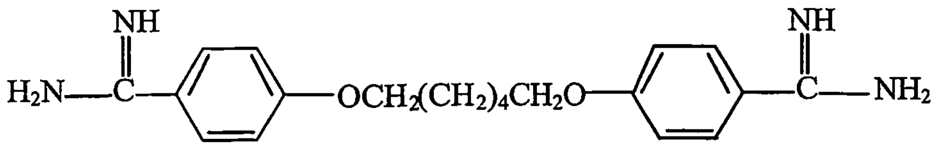

These aromatic diamines are referred to as 4,4'-[1,6-Hexanediylbis(oxy)]bisbenzenecarboximidamide; 4,4'-(hexamethylenedioxy)dibenzamidine; and 4,4'-diamidino-α,ω-diphenoxyhexane. The most popular employed form of hexamidine is the general category of hexmidine salts, which include acetate, salicylate, lactate, gluconate, tartarate, titrate, phosphate, borate, nitrate, sulfate, and hydrochloride salts of hexamidine. Specific nonlimiting examples of hexamidine salts include hexamidine isethionate, hexamidine diisethionate, hexamidine hydrochloride, hexamidine gluconate, and mixtures thereof. Hexamidine isethionate and hexamidine diisethionate are β-hydroxyethane sulfonate salts of hexamidine which are preferred for use herein as a skin treatment agent in the prevention and/or treatment of skin disorders. Hexamidine diisethionate is the most preferred hexamidine compound suitable for use as the skin treatment agent herein.

Hexamidine compounds are known as effective skin treatment agents that can control microbial growth that can lead to irritating and itching skin disorders. Therefore, these skin treatment agents are often referred to as antimicrobial

agents. As used herein the term "antimicrobial agents" refer to materials which function to destroy or suppress the growth or metabolism of microbes, and include the general classification of antibacterial, antifungal, antiprotozoal, antiparasitic, and antiviral agents.

It has been found, however, that a low concentration (about 0.1% or less by weight) of hexamidine provides for improved reduction and/or prevention of skin irritating infections, especially when a low amount of hexamidine is combined with a low concentration of other antimicrobial agents such as zinc oxide and niacinamide. This combination of hexamidine, zinc oxide, and niacinamide can be administered topically and internally at a total concentration less than an effective amount of an applied dosage of these individual compounds. As used herein the term "effective amount" refers to an amount with provides a therapeutic benefit with minimal or no adverse reaction in the reduction and/or prevention of any noticeable or unacceptable skin abnormality which causes irritating, acute, or chronic symptoms including itching and inflammation.

Commercially available hexamidine compounds suitable for use as a skin treatment agent herein include hexamidine diisethionate which is available from Laboratories Serolobilogiques (Pulnoy, France) and the Cognis Incorporation (Cincinnati, Ohio) under the tradename ELASTAB HP100.

Other aromatic diamines are also suitable for use as a skin treatment agent herein. Such compounds include butamidine and derivatives thereof including butamidine isethionate; pentamidine and derivatives thereof including pentamidine isethionate and pentamidine hydrochloride; dibromopropamidine and derivatives thereof including dibromopropamidine isethionate; stilbamidine and derivatives thereof including hydroxystilbamidine, stilbamidine dihydrochloride, and stilbamidine isethionate; diaminodiamidines and derivatives thereof; and mixtures thereof.

## Zinc Oxide

The skin care compositions comprise zinc oxide skin treatment agent at concentrations ranging from 0.001 % to 30%, preferably from 0.005% to 10%, more preferably from 0.005% to 2%, most preferably from 0.01% to 1% by weight of the composition. The zinc oxide skin treatment agent can be included in the compositions as an individual zinc oxide compound or a combination of zinc oxides, provided that the individual or combined zinc oxide can readily combine with the hexamidine and niacinamide skin treatment agents to provide antimicrobial benefits.

The zinc oxide skin treatment agent suitable for use herein include those inorganic white and yellowish-white powders that conform to the formula ZnO, and that are more fully described in The Merck Index, Eleventh Edition, entry 10050, p. 1599 (1989).

Commercially available zinc oxides include the white zinc oxide powders sold under the tradename ULTRAFINE 350 which is commercially available from the Kobo Corporation located in South Plainfield, New Jersey. Other suitable zinc oxide materials include a premix of zinc oxide and a dispersing agent such as polyhydroxystearic acid wherein this premix is available from the Uniqema Incorporation (Wilimington, Delaware) under the tradename Arlecel® P100; and a premix of zinc oxide and an isononyl isononanoate dispersing agent which is available from the Ikeda Incorporation (Island Park, New York) under the tradename Salacos® 99.

## Niacinamide

The skin care compositions comprise niacinamide skin treatment agent as an individual niacinamide or as a combination of niacinamides at a total niacinamide concentration ranging from 0.01% to 10%, preferably from 0.05% to 5%, more preferably from 0.2% to 2% by weight of the skin care composition. The niacinamide skin treatment agent provides for skin conditioning benefits as well as providing for increased efficacy of the skin treatment agents in controlling skin disorders.

Nonlimiting examples of niacinamide skin treatment agents suitable for use in the skin care compositions of the present invention include those niacinamide compounds that are amide derivatives of nicotinic acid, and that generally conform to the following formula:

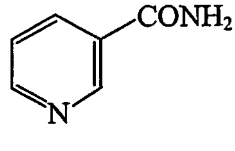

Niacinamide and nicotinic acid are also known as Vitamin B3 and Vitamin B5, whereas niacinamide is the commonly used active form. Niacinamide derivatives including salt derivatives are also suitable for use herein as a skin treatment agent. Nonlimiting specific examples of suitable niacinamide derivatives include nicotinuric acid and nicotinyl

hydroxamic acid.

The niacinamide skin treatment agent can also be included in the composition as acidified niacinamide compounds. The process of acidifying niacinamide compounds is within the gambit of those skilled in the art, wherein one such technique involves dissolving niacinamide in an alcohol solution, adding while stirring an equal molar amount of a fatty acid such as stearic acid (e.g., mixing 1 part niacinamide to 2.4 parts stearic acid), and then air drying the mixture until the alcohol evaporates. A suitable stearic acid compound that can be used in the process of acidifying niacinamide is stearic acid sold under the tradename Emersol® 150 which is available from the Cognis Corporation.

Examples of the above niacinamide compounds are well known in the art and are commercially available from a number of sources, for example, the Sigma Chemical Company (St Louis, Missouri); ICN Biomedicals, Incorporation (Irvin, California); Aldrich Chemical Company (Milwaukee, Wisconsin); and Em Industries HHN (Hawthorne, New York).

## Carrier

The skin care compositions comprise a carrier for the skin treatment agents. The carrier can be included in the compositions as an individual carrier or a combination of carrier ingredients, provided that the total carrier concentration is sufficient to provide transfer and/or migration of the skin treatment agents onto the skin. The carrier can be a liquid, solid, or semisolid carrier material, or a combination of these materials, provided that the resultant carrier forms a homogenous mixture or solution at selected processing temperatures for the resultant carrier system and at processing temperatures for combining the carrier with the skin treatment agents in formulating the skin care compositions herein. Processing temperatures for the carrier system typically range from about 60°C to about 90°C, more typically from about 70°C to about 85°C, even more typically from about 70°C to about 80°C.

The skin care compositions of the present invention typically comprise the carrier at a total carrier concentration ranging from 60% to 99.9%, preferably from 70% to 98%, more preferably from 80% to 97% by weight of the skin care composition. Suitable carrier compounds include petroleum-based hydrocarbons having from 4 to 32 carbon atoms, fatty alcohols having from 12 to 24 carbon atoms, polysiloxane compounds, fatty acid esters, alkyl ethoxylates, lower alcohols having from 1 to 6 carbon atoms, low molecular weight glycols and polyols, fatty alcohol ethers having from 12 to 28 carbon atoms in their fatty chain, lanolin and its derivatives, glyceride and its derivatives including acetoglycerides and ethoxylated glycerides of C12-C28 fatty acids, and mixtures thereof.

Nonlimiting examples of suitable petroleum-based hydrocarbons having from 4 to 32 carbon atoms include mineral oil, petrolatum, isoparaffins, various other branched chained hydrocarbons, and combinations thereof. Mineral oil is also known as "liquid petrolatum", and usually refers to less viscous mixtures of hydrocarbons having from 16 to 20 carbon atoms. Petrolatum is also known as "mineral wax", "petroleum jelly", and "mineral jelly", and usually refers to more viscous mixtures of hydrocarbons having from 16 to 32 carbon atoms. Mineral oil and petrolatum are the preferred hydrocarbons suitable for use as a carrier herein. Petrolatum is most preferred, and an example of commercially available petrolatum include petrolatum sold as Protopet® 1S which is available from the Witco Corporation located in Greenwich, Connecticut.

Other suitable hydrocarbons having from 4 to 30 carbon atoms include, but are not limited to, the isoparaffins available from Exxon Chemical Corporation (Baytown, Texas) as Isopar C (C7-C8 Isoparaffin), Isopar E (C8-C9 Isoparaffin), Isopar G (C10-C11 Isoparaffin), Isopar H (C11-C12 Isoparaffin), Isopar L (C11-C13 Isoparaffin), Isopar M (C13-C14 Isoparaffin), and combinations thereof. Other nonlimiting examples of suitable paraffins include the Norpar series available from Exxon Chemical Company as Norpar 12, Norpar 13, and Norpar 15.

Other suitable hydrocarbons having from 4 to 30 carbon atoms include, but are not limited to, branched chain hydrocarbons such as Permethyl 99A (isododecane), Permethyl 102A (isoeicosane), Permethyl 101A (isohexadecane), and combinations thereof. The permethyl series are available from Preperse Corporation located in South Plainfield, New Jersey. Other nonlimiting examples of suitable branched chained hydrocarbons include petroleum distillates such as those available from Phillips Chemical as Soltrol 130, Soltrol 170, and those available from Shell as Shell Sol 70, Shell Sol 71, and Shell Sol 2033, and combinations thereof.

Nonlimiting examples of suitable fatty alcohols having from 12 to 24 carbon atoms include saturated, unsubstituted, monohydric alcohols or combinations thereof, which have a melting point less than 110°C, preferably from 45°C to 110°C. Specific examples of fatty alcohol carriers for use in the skin care compositions of the present invention include, but are not limited to, cetyl alcohol, stearyl alcohol, cetearyl alcohol, behenyl alcohol, arachidyl alcohol, lignocaryl alcohol, and combinations thereof. Cetearyl alcohol and behenyl alcohol are preferred. Examples of commercially available preferred cetearyl alcohol is Stenol 1822 and behenyl alcohol is Lanette 22, both of which are available from the Cognis Corporation located in Cincinnati, Ohio. The fatty alcohol carriers help to provide skin conditioning benefits such as softness, smoothness and moisturization benefits.

Nonlimiting examples of suitable polysiloxane compounds include modified or organofunctional silicones such as polyalkylsiloxanes, polyalkylarylsiloxanes, polyestersiloxanes, polyethersiloxanes copolymers, polyfluorosiloxanes, polyaminosiloxanes, and combinations thereof. These polysiloxanes carriers are typically liquid under ambient conditions,

and have a preferred viscosity ranging from 1 centipoise to 100,000 centipoise (as measured at 37°C using a glass viscometer), more preferably from 5 centipoise to 5,000 centipoise, even more preferably from 5 centipoise to 2,000 centipoise, and most preferably from 100 centipoise to 1000 centipoise. Polysiloxane carriers having a viscosity greater than 100,000 centipoise, particularly from 100,000 centipoise to 20,000,000 centipoise are also suitable for use herein provided that the polysiloxane carriers are liquids or included in a liquid solution by addition of the polysiloxane in a solution of a surfactant or other suitable solvent such as hexane. Polysiloxane carriers are known in the art, some examples of which are described in Cosmetics, Science and Technology, Vol. 1, pp. 27-104, (M. Balsam and E. Sagarin ed. 1972); and U.S. Patent No. 5,509,282 issued to Ampulski et al. on October 22, 1991. Specific examples of polysiloxane carriers suitable for use herein include a polyalkylarylsiloxane commercially available as Dow Corning® 556 Fluid (phenyldimethicone); and polyalkylsiloxanes commercially available as Dow Corning 2502® Fluid (cetyl functional dimethicone) and Dow Corning® 2503 Fluid (stearyl functional dimethicone); all of which are available from the Dow Coming Corporation located in Midland, Michigan.

Nonlimiting examples of suitable fatty acid esters include those fatty acid esters derived from a mixture of C12-C28 fatty acids and short chain (C1-C8, preferably C1-C3) monohydric alcohols preferably from a mixture of C16-C24 saturated fatty acids and short chain (C1-C8, preferably C1-C3) monohydric alcohols. Representative examples of such esters include methyl palmitate, methyl stearate, isopropyl laurate, isopropyl myristate, isopropyl palmitate, ethylhexyl palmitate, and mixtures thereof. Suitable fatty acid esters can also be derived from esters of longer chain fatty alcohols (C12-C28, preferably C12-C16) and shorter chain fatty acids such as lactic acid, specific examples of which include lauryl lactate and cetyl lactate. The fatty acid esters also provide for skin conditioning benefits such as moisturization and smoothness to the skin.

Nonlimiting examples of suitable alkyl ethoxylates include C12-C22 fatty alcohol ethoxylates having an average degree of ethoxylation of from 2 to 30. Preferably, the fatty alcohol ethoxylate is selected from the group consisting of lauryl, cetyl, stearyl, and behenyl ethoxylates, and mixtures thereof, having an average degree of ethoxylation ranging from 2 to 23. Representative examples of such alkyl ethoxylates include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10), steareth-2 (a stearyl alcohol ethoxylate having an average degree of ethoxylation of 2), steareth-10 (a stearyl alcohol ethoxylate having an average degree of ethoxylation of 10), beheneth-10 (a behenyl alcohol ethoxylate having a degree of ethoxylation of 10), and mixtures thereof. When the carrier includes these alkyl ethoxylates in combination with petrolatum the weight ratio of alkyl ethoxylate to petrolatum is typically from about 1:1 to about 1:5, preferably from about 1:2 to about 1:4. Specific examples of commercially available C12-C22 fatty alcohol ethoxylates include a beheneth-10 fatty alcohol ethoxylate commercially available as Mergital® B10 from the Cognis Corporation, and a steareth-2 fatty alcohol ethoxylate commercially available as Brij® 762 from the Uniqema Corporation.

Nonlimiting examples of suitable lower alcohols having from about 1 to about 6 carbon atoms include ethanol, isopropanol, butanediol, 1,2,4-butanetriol, 1,2 hexanediol, ether propanol, and mixtures thereof.

Nonlimiting examples of suitable low molecular weight glycols and polyols include ethylene glycol, polyethylene glycol (e.g., Molecular Weight 200-600 g/mole), butylene glycol, propylene glycol, polypropylene glycol (e.g., Molecular Weight 425-2025 g/mole), and mixtures thereof.

Preferably, the carrier comprises a combination of one or more petroleum-based hydrocarbons and one or more fatty alcohols described hereinabove. When one or more petroleum-based hydrocarbons having from about 4 to about 32 carbon atoms are used in combination with one or more fatty alcohols having from about 12 to about 22 carbon atoms, the petroleum-based hydrocarbons are included at total concentrations ranging from 20% to 99%, preferably from 30% to 85%, more preferably from 40% to 80% by weight of the skin care composition; wherein the fatty alcohols are included at total concentrations ranging from 0.2% to 65%, preferably from 1% to 50%, more preferably from 2% to 40% by weight of the skin care composition. The carrier can comprise other ingredients in addition to the petroleum-based hydrocarbons and fatty alcohols, such ingredients include fumed silica. It is believed that a petroleum-based carrier system comprising C4-C32 hydrocarbons, C12-C22 fatty alcohols, and fumed silica provides a homogeneous mixture of the carrier, skin treatment agents, and any optional ingredients wherein this homogeneous mixture ensures sufficient contact between the skin and skin treatment agents to result in effective prevention and treatment of skin disorders.

The fumed silica suitable for inclusion in the preferred petroleum-based carrier system, or with any other carrier described herein, includes colloidal pyrogenic silica pigments which are sold under the Cab-O-Sil® tradename, and which are commercially available from the Cabot Corporation located in Tuscola, Illinois. These colloidal pyrogenic silica pigments are submicroscopic particulated pyrogenic silica pigments having mean particle sizes ranging from about 0.1 microns to about 100 microns. Specific examples of commercially available Cab-O-Sil® silica pigments include Cab-O-Sil® TS-720 (a polydimethylsiloxane treated fumed silica), Cab-O-Sil® TS-530 (a trimethyl silanized fumed silica), and Cab-O-Sil® TS-610 (a dimethyldisilanized fumed silica). The fumed silica provides the skin care compositions with desired viscosity or thickening properties, and is typically included at concentrations ranging from 0.01% to 15%, preferably from 0.1% to 10%, more preferably from about 1% to 5% by weight of the skin care composition.

The fumed silica can be used alone or in combination with other optional viscosity or thickening agents such as talc, bentonites including treated bentonites, hectorites including treated hectorites, calcium silicates including treated calcium silicates, magnesium silicates, magnesium aluminum silicates, zinc stearates, sorbitol, colloidal silicone dioxides, spermaceti, carnuba wax, beeswax, candelilla wax, paraffin wax, microcrystalline wax, castrol wax, ceresin, esparto, ouricuri, rezowax, polyethylene wax, C12-C24 fatty acids, polyhydroxy fatty acid esters, polyhydroxy fatty acid amides, polymethacrylate polymers, polymethacrylate and styrene copolymers, and combinations thereof. These other optional viscosity modifying or thickening agents are also included at total concentrations ranging from about 0.01% to about 15% by weight of the skin care composition. A nonlimiting specific example of another suitable viscosity or thickening agent include bentonite sold as Bentone® 38 which is available from the Rheox Incorporation.

It is preferable that the carrier's surface energy be similar to the body contacting surface. For example, in cases where the topsheet is hydrophobic the carrier will preferably be hydrophobic. Further, under these circumstances it is preferable that the lotion composition of the present invention comprise no surfactant. Therefore, in a preferred embodiment of the present invention the lotion has a level of hydrophobicity at least as great as that of the topsheet, and the hydrophobicity of the lotion is primarily due to the lack of a surfactant component. If, under some condition, there is a need to raise the wettability of the hydrophobic carrier one may optionally add a wetting agent such as polyoxyethylene alkyl ethers, alkyl ethoxylates, alkylethoxylated amines, polyethylene glycol esters, and/or sorbitan fatty acid esters generally having a low degree of ethoxylation and HLB values below about 7. Suitable additives should be miscible with the carrier so as to form a homogenous mixture. Because of possible skin sensitivity of those using the catamenial device of the present invention, these wetting agents should also be relatively mild and non-irritating to the skin. Typically, these wetting agents are nonionic to be not only non-irritating to the skin, but also to avoid other undesirable effects on any underlying tissue laminate structure, e.g., reductions in tensile strength. Suitable wetting agents will typically have HLB values below 10, preferably below 9, more preferably below 8, and even more preferably below 7.

Non-limiting specific examples of a suitable wetting agents includes nonyl phenol or polyoxyethylene nonyl phenyl ether (2 degrees of ethoxylation; HLB of 5.7), octyl phenol or polyoxyethylene octyl phenyl ether (1 degree of ethoxylation; HLB of 3.5), stearyl alcohol or polyoxyethylene stearyl ether (2 degrees of ethoxylation; HLB of 4.9), stearyl amine or polyoxyethylene stearyl amine (2 degrees of ethoxylation; HLB of 4.9), polyethylene glycol 200 dilaurate (HLB 5.9), polyethylene glycol 200 distearate (HLB 4.8), sorbitan monostearate ('Span 60' having HLB 4.7), sorbitan tristearate ('Span 65' having HLB 2.1), sorbitan monooleate ('Span 80' having HLB 4.3), sorbitan trioleate ('Span 85' having HLB 1.8), each of which are available form Cell Chemical Company (Inchon, Korea) or Uniqema (New Castle, Delaware, USA).

The amount of wetting agent required to increase the wettability of the lotion composition to a desired level will depend upon its HLB value and HLB level of the carrier used, and like factors. The lotion composition can comprise from about 1 to about 50% of the wetting agent when needed to increase the wettability properties of the composition. Preferably, the lotion composition comprises from about 1 to about 25%, most preferably from about 10 to about 20%, of the wetting agent when needed to increase wettability.

In cases where the lotion that is applied is hydrophobic and the body facing material is also hydrophobic it may be preferable to apply the lotion in a non-uniform manner, such as stripes, dots, etc. in order to reduce the possibility of rectal secretions or blood from being absorbed by the pad and staining the wearer's garments.

In come cases it is also preferable that the composition of the carrier reduces the coefficient of friction of the body facing material.

## Optional Components

In addition to the essential skin treatment and carrier components described herein, the skin care compositions of the present invention may further comprise one or more optional components known or otherwise effective for use in skin care compositions, provided that the optional components are physically and chemically compatible with the essential skin treatment and carrier components, or do not otherwise unduly impair product stability, aesthetics, or performance. Such optional components are typically included at concentrations ranging from 0.001% to 20% by weight of the compositions, preferably 0.00 1 % to 15%, and more preferably from 0.0 1 % to 10% and include materials such as water, skin conditioning agents, perfumes, deodorants, opacifiers, astringents, preservatives, anti-microbial agents, neosporin, bacitracin, corticosteroids, pramoxine HCl, emulsifying agents, film formers, anti-itch, stabilizers, proteins, lecithin, urea, colloidal oatmeal, pH control agents, and other monographed materials that are deemed safe by the U.S. Food and Drug Administration (FDA) under 21 C.F.R. §347 for use on human skin. Other non-limiting examples of skin care agents are described in U.S. Pat. No. 6,153,209 issued to Vega.

Other optional components for use in the skin care compositions of the present invention include fats or oils, or essential oils. These oils can be present at concentrations ranging from about 0.0001% to 10% by weight of the compositions, and include materials such as Anise Oil, Balm Mint Oil, Basil Oil, Bee Balm Oil, Bergamot Oil, Birch Oil, Bitter Almond Oil, Bitter Orange Oil, Calendula Oil, California Nutmeg Oil, Caraway Oil, 345 Cardamom Oil, Chamomile

Oil, Cinnamon Oil, Clary Oil, Cloveleaf Oil, Clove Oil, CorianderOil, Cypress Oil, Eucalyptus Oil, Fennel Oil, Gardenia Oil, Geranium Oil, Ginger Oil, Grapefruit Oil, Hops Oil, Hyptis Oil, Indigo Bush Oil, Jasmine Oil, Juniper Oil, Kiwi Oil, Laurel Oil, Lavender Oil, Lemongrass Oil, Lemon Oil, Linden Oil, Lovage Oil, Mandarin Orange Oil, Matricaria Oil, Musk Rose Oil, Nutmeg Oil, Olibanurn, Orange Flower Oil, Orange Oil, 350 Patchouli Oil, Pennyroyal Oil, Peppermint Oil, Pine Oil, Pine Tar Oil, Rose Hips Oil, Rosemary Oil, Rose Oil, Rue Oil, Sage Oil, Sambucus Oil, Sandalwood Oil, Sassafras Oil, Silver Fir Oil, Spearmint Oil, Sweet Marjoram Oil, Sweet Violet Oil, Tar Oil, Tea Tree Oil, Thyme Oil, Wild Mint Oil, Yarrow Oil, Ylang Ylang Oil, Apricot Kernel Oil, Avocado Oil, Babassu Oil, Borage Seed Oil, Butter, C12-C1. Acid Triglyceride, Camellia Oil, Canola Oil, Caprylic/Capric/Lauric Triglyceride, Caprylic/Capric/Linoleic Triglyceride, Caprylic/Capric/Stearic Triglyceride, Caprylic/Capric305 Triglyceride, Carrot Oil, Cashew Nut Oil, Castor Oil, Cherry Pit Oil, Chia Oil, Cocoa Butter, Coconut Oil, Cod Liver Oil, Corn Germ Oil, Corn Oil, Cottonseed Oil, C10-C1 Triglycerides, Egg Oil, Epoxidized Soybean Oil, Evening Primrose Oil, Glyceryl Triacetyl Hydroxystearate, Glyceryl Triacetyl Ricinoleate, Glycosphingolipids, Grape Seed Oil, Hazelnut Oil, Human Placental Upids, Hybrid Safflower Oil, Hybrid Sunflower Seed Oil, Hydrogenated Castor Oil, 310 Hydrogenated Castor Oil Laurate, Hydrogenated Coconut Oil, Hydrogenated Cottonseed Oil, Hydrogenated C2-C1 Triglycerides, Hydrogenated Fish Oil, Hydrogenated Lard, Hydrogenated Menhaden Oil, Hydrogenated Mink Oil, Hydrogenated Orange Roughy Oil, Hydrogenated Palm Kernel Oil, Hydrogenated Palm Oil, Hydrogenated Peanut Oil, Hydrogenated Shark Liver Oil, Hydrogenated Soybean Oil, Hydrogenated Tallow, 315 Hydrogenated Vegetable Oil, Lard, Lauric/Palmitic/Oleic Triglyceride, Lanolin and Lanolin derivatives, Lesquerella Oil, Linseed Oil, Macadamia Nut Oil, Maleated Soybean Oil, Meadowfoarn Seed Oil, Menhaden Oil, Mink Oil, Moringa Oil, Mortierella Oil, Neatsfoot Oil, Oleic/Linoleic Triglyceride, Oleic/Paimitic/Lauric/Myristic/Linoleic Triglyceride, Oleostearine, Olive Husk Oil, Olive Oil, Ornental Lipids, Orange Roughy Oil, Palm Kernel Oil, Palm Oil, 320 Peach Kernel Oil, Peanut Oil, Pengawar Djambi Oil, Pentadesma Butter, Phospholipids, Pistachio Nut Oil, Placental Lipids, Rapeseed Oil, Rice Bran Oil, Safflower Oil, Sesame Oil, Shark Liver Oil, Shea Butter, Soybean Oil, Sphingolipids, Sunflower Seed Oil, Sweet Almond Oil, Tall Oil, Tallow, Tribehenin, Tricaprin, Tricaprylin, Triheptanoin, C10 Fatty Acids: Arachidic Acid, Arachidonic Acid, Behenic Acid, Capric Acid, Caproic Acid, 330 Caprylic Acid, Coconut Acid, Corn Acid, Cottonseed Acid, Hydrogenated Coconut Acid, Hydrogenated Menhaden Acid, Hydrogenated Tallow Acid, Hydroxystearic Acid, Isostearic Acid, Lauric Acid, Linoleic Acid, Linolenic Acid, Linseed Acid, Myristic Acid, Oleic Acid, Palmitic Acid, Palm Kernel Acid, Pelargonic Acid, Ricinoleic Acid, Soy Acid, Stearic Acid, Tall Oil Acid, Tallow Acid, Undecanoic Acid, Undecylenic Acid, Wheat Germ Acid, and the like, as well as mixtures thereof.

Other optional components for use in the skin care compositions of the present invention include extracted botanical actives containing the chemically "active" components, of various plants and plant substances. Extracted botanical actives can include any water-soluble or oil-soluble active extracted from a particular plant. Examples of suitable extracted botanical actives are actives extracted from echinacea, yucca glauca, willow herb, basil leaves, aloe, Turkish oregano, carrot root, grapefruit fruit, fennel fruit,rosemary, thyme, blueberry, bell pepper, black tea, blackberry, blackcurrant fruit, Chinese tea, coffee seed, dandelion root, date palm fruit, gingko leaf, green tea polyphenols (i.e. including epicatechin gallate and epigallocatechin 3-O-gallate), hawthorn berries, licorice, oolong tea,sage, strawberry, sweet pea, tomato, vanilla fruit, neohesperidin, quercetin, rutin, morin, myricetin, chlorogenic acid, glutathione, glycyrrhizin, absinthe, arnica, centella asiatica, chamomile, comfrey, cornflower, horse chestnut, ivy (Herdera helix), magnolia, mimosa, oat extract, pansey, scullcap, seabuckthorn, white nettle, witch hazel and any combinations thereof. Particular benefits have been observed with compositions including echinacea, yucca glauca, green tea, black tea, oolong tea, Chinese tea, chamomile, aloe, and willow herb. The compositions of the invention can include from 0.1 to 10 percent by weight of one or more extracted botanical actives. More specifically, the compositions can include from 0.2 to 7 percent by weight of one or more extracted botanical actives. Even more specifically, the compositions include from 1 to 5 percent by weight of extracted botanical actives.). In particular aspects, the extracted botanical actives can be at least a minimum of 0.1 percent by weight. The extracted botanical actives can alternatively be at least 0.5 percent, and optionally, can be at least about 1 percent to provide improved performance. In other aspects, the extracted botanical actives can be not more than a maximum of 10 percent by weight. The extracted botanical actives can alternatively be not more than 8 percent, and optionally, can be not more than 5 percent. Botanicals are primarily extracts of the plants from which they originate and botanicals are available from suppliers as part of a composition that also contains an extracting solvent. Amounts of the botanicals in the compositions of the invention in terms of active component (not extract)may range from 0.000001 to 10% by weight. Desirably, the amount of active botanical is from 0.00001 to 5% and more desirably from 0.0001 to 1% by weight of the composition. Further, it is also desirable that the amount of active botanical is from 0.0001 to 0.5% of the composition and more desirably from 0.001 to 0.1% by weight of the composition. Sometimes it is necessary for the compositions of the invention to include additional components that can be used to emulsify or suspend the extracted botanical active with the rest of the composition. If the extracted botanical active is not properly incorporated into the composition, it may not have the bioavailability to provide benefits to the skin. In addition to modifications to the formulation, extracted botanical actives can also be better incorporated through the use of processing techniques. For compositions of the invention including Echinacea and chamomile, it may be necessary to add an emulsifying agent such as sorbitan monooleate, sorbitan sesquioleate, sorbitan trioleate and glycerol monooleate.

Other optional components for use in the skin care treatment of the present invention include non-modified hydrophilic and modified hydrophobic clays including smectite, montmorillonite, bentonite, beidellite, hectorite, saponite and stevensite, and their synthetically made counterparts such as laponite for example.

The skin care compositions of the present invention preferably comprise optional skin conditioning agents such as panthenol, glycerine, and chamomile oil which are described in detail hereinbelow.

**Panthenol**

The skin care compositions can comprise the preferred optional panthenol skin conditioning agent at concentrations ranging from 0.001% to 10%, preferably from 0.005% to 5%, more preferably from 0.05% to 1% by weight of the skin care composition. The optional panthenol skin conditioning agent provides for skin emolliency benefits that can leave the skin feeling smooth, soothing, and soft during and after interaction of the skin tissues with the skin treatment agents.

The skin care compositions of the present invention can include an individual panthenol compound or a mixture of panthenol compounds, provided that the total concentration of panthenol included in the composition provides for the above-described skin conditioning benefits, wherein such total concentrations generally range from 0.001% to 10% by weight of the composition.

Nonlimiting examples of preferred optional panthenol skin conditioning agents suitable for use in the skin care compositions of the present invention include those panthenol compounds which are alcohol derivatives of pantothenic acid. Pantothenic acid is a member of the B complex family and is often referred to as Vitamin B3. Like pantothenic acid, the panthenol alcohol derivatives of this acid can exist as stereoisomers, for example, the D(+) form, the L(-) form, the racemate, and mixtures of the D(+) and L(-) forms. Therefore, the term "panthenol" as used herein refers to the D(+) form, the L(-) form, the racemate, and mixtures of the D(+) and L(-) forms, unless otherwise specified. Specific examples of panthenol include, but are not limited to, D-panthenol (a.k.a. dexpanthenol), and dl-panthenol. Panthenol is more fully described in The Merck Index, Eleventh Edition, entry 2924, p. 464 (1989), which description is incorporated herein by reference.

Examples of commercially available panthenol include D-panthenol which is available from Roche Vitamins Incorporation (Nutley, New Jersey), a subsidiary of F. Hoffman LaRoche, Ltd.

**Glycerine**

The skin care compositions can comprise the preferred optional glycerine skin conditioning agent at concentrations ranging from 0.01% to 10%, preferably from 0.02% to 5%, more preferably from 0.05% to 2% by weight of the skin care composition. The optional glycerine skin conditioning agent also provides for skin emolliency benefits such as smooth, soothing, and soft feeling skin, as well as being a dispersing agent for the niacinamide skin treatment agent.

Glycerine is a C3 monohydric alcohol that is also referred to as glycerol and 1,2,3-propanetriol. Glycerine derivatives are also suitable for use as an optional skin conditioning agent herein wherein such derivatives include polyglycerols having from about 2 to about 16 repeating glycerol moieties. A specific example of a suitable glycerine skin conditioning agent is Glycerine, USP Kosher® which is commercially available from the Procter & Gamble Company located in Cincinnati, Ohio.

**Chamomile**

The skin care compositions can comprise the preferred optional chamomile oil at concentrations ranging from 0.0001% to 10%, preferably from 0.001% to 5%, more preferably from 0.005% to 2% by weight of the skin care composition. The optional chamomile oil skin conditioning agent also provides for skin benefits such as soothing. Chamomile oil is commonly prepared as an oil extract of chamomile flowers. An example of a commercially available chamomile oil include Phytoconcentrol Chamomile which is available from Dragoco Incorporation (Totowa, New Jersey).

**Methods of Treating the Skin**

The present invention also relates to methods of treating the perianal skin with the skin care compositions described herein. Generally, a safe and effective amount of the skin care composition is applied to a hemorrhoid treatment pad described herein wherein such safe and effective amounts include applying from 0.0155 g/m2 (0.01 mg/in2) to 310 g/m2 (200 mg/in2) preferably from 0.155 g/m2 (0.1 mg/in2) to 155 g/m2 (100 mg/in2), more preferably 0.5 g/m2 (0.003 mg/in2) to 93 g/m2 (60 mg/in2), of the skin care composition to the absorbent article.

Typically, a safe and effective amount of the skin care compositions of the present invention is applied to the

body facing surface of the hemorrhoid treatment pad such that at least 0.0015 g/cm2 (0.001 mg/in2) to 310 g/cm2 (200 mg/in2), preferably from 0.006 g/cm2 (0.004 mg/in2) to 155 g/cm2 (100 mg/in2), more preferably from 0.05 g/cm2 (0.03 mg/in2) to 62 g/cm2 (40 mg/in2), of the composition is transferred to the skin during a single use of an absorbent article which can be, for example, about a two hour period. Hemorrhoid treatment pads can be changed every two to five hours during the day and once for overnight use, resulting in at least a safe and effective amount of from 0.0045 g/cm2 (0.003 mg/in2) to 1860 g/cm2 (1200 mg/in2), preferably from 0.018 g/cm2 (0.012 mg/in2) to 930 g/cm2 (600 mg/in2), more preferably from 0.15 g/cm2 (0.09 mg/in2) to 372 g/cm2 (240 mg/in2), of the skin care composition being administered within a one day interval (24 hour period). The transfer of the skin care compositions of the present invention onto a wearer's skin via a hemorrhoid treatment pad described herein can occur for one day, several days, weeks, months, or years at appropriate intervals provided that safe and effective amounts of the skin care compositions are administered to deliver the skin treatment benefits described herein.

The skin care compositions of the present invention can be applied to the hemorrhoid treatment pads by any known or otherwise effective technique. Nonlimiting examples of methods of applying the skin care compositions onto an absorbent article include spraying, printing (e.g., flexographic printing), coating (e.g., contact slot coating and gravure coating), extrusion, or combinations of these application techniques. The application of the skin care compositions onto a hemorrhoid treatment pad facilitates the transfer or migration of the skin care compositions onto the skin for administration and/or deposition of the skin care compositions, resulting in a safe and effective amount of the compositions being applied for improved prevention and reduction of skin disorders. Therefore, the safe and effective amount of the skin care composition that will transfer or migrate to the skin will depend on factors such as the type of skin care composition that is applied, the portion of the body contacting surface where the skin care composition is applied, and the type of absorbent article used to administer the skin care composition.

Any suitable method can be used in determining the amount of a skin care composition described herein that is transferred to the skin of a wearer during use of a hemorrhoid pad containing the composition. An example of specific methods for the calculation of transfer amounts of skin care compositions include Gas Chromatographic and other quantitative analytical procedures that involve the analysis of in vivo skin analog materials. A suitable Gas Chromatographic procedure is more fully described in WO 99/45973, Donald C. Roe et al, published September 16, 1999.

## Method of Manufacture

The skin care compositions of the present invention may be prepared by any known or otherwise effective technique, suitable for providing a skin care composition comprising the essential skin treatment agents defined herein.

In general, the skin care compositions are prepared by first making a carrier system comprising suitable carriers such as petrolatum, behenyl alcohol, and beheneth-10 in combination with a fumed silica thickening agent. Next, a mixture comprising the skin treatment agents and any optional ingredients such as optional skin conditioning agents are added to the carrier system at a melt mix temperature of 80°C. Although the carrier system, skin treatment agents, and any optional ingredients are typically processed at a temperature of 80°C, these materials can be processed at temperatures ranging from 60°C to 90°C, preferably from 70°C to 90°C. The resultant skin care composition can be subsequently applied to the topsheet 42 using a contact applicator such as a Nordsen EP 11-12-02.

The skin care compositions of the present invention are prepared such that the compositions can be applied to a hemorrhoid pad to result in safe and effective amounts of the compositions being transferred onto the skin of a wearer of the absorbent article. Therefore, the skin care compositions preferably have a product consistency such that they are relatively immobile and localized on the wearer-contacting surface of the absorbent article at ambient conditions, are readily transferable to the wearer at body temperature, and yet are not completely liquid under extreme storage conditions. In other words, the skin care compositions are solids or semisolids at ambient conditions (about 25°C) and/or body temperature (about 37°C) so that the compositions are easily transferred onto the skin by way of normal contact, wearer motion, and/or body heat. The consistency of the skin care compositions can be measured according to ASTM D5 test method which involves the use of a penetrometer to measure consistency. Typically, the skin care compositions of the present invention have a consistency of from 10 to 300, preferably from about 20 to about 250, more preferably from 30 to 200, as measured at 40°C according to the test procedure outlined in ASTM D5 test method.

The solid or semisolid consistency of the skin care compositions provide for relatively low levels of the compositions to be applied to the absorbent articles to impart the desired skin care benefits. By "semisolid" is meant that the compositions have a rheology typical of pseudoplastic or plastic liquids such that the compositions remain relatively stationary in a desired location on the absorbent article, and do not have a tendency to flow or migrate to undesired locations of the article. The solid skin care compositions of the present invention likewise can remain in a particular location and not flow or migrate to undesired locations of the article. These solid and semisolid skin care compositions have viscosities high enough to keep the compositions localized on an intended location of the article, but not so high as to impede transfer to the wearer's skin. Typically, final products of solid and semisolid skin care compositions have viscosities ranging from 1.0 x 106 centipoise to 1.0 x 1010 centipoise under shear stress conditions of 3 x 103 dynes/cm2

at 40°C (the shear stress applied to the compositions while the absorbent article is in storage or transported at temperature conditions of about 40°C).

However, the solid and semisolid skin care compositions can be made flowable for transfer or migration of the compositions onto the skin by applying shear stress that results in deformation of the compositions. The shear stress applied at least once during wear of the absorbent article under temperature conditions of about 40°C is typically at 1.0 x106 dynes/cm2, and this shear stress can result in the skin care compositions having a viscosity of from about 1.0x101 centipoise to 1.0x 105 centipoise. It is believed that the skin care compositions achieve the lower viscosity values under applied shear stress due to the fact that, while the compositions contain solid components, they also contain liquid materials. During wear of an absorbent article described herein, it is desirable to achieve a low viscosity for obtaining sufficient lubrication between the wearer's skin and the body contacting surface of the article to result in effective transfer of the skin care composition onto the wearer's skin. Viscosity at various shear stress can be measured using rheometers known in the art such as the Rheometer SR-2000 available from Rheometrics Incorporation.

The skin care compositions are preferably applied to a hemorrhoid treatment pad for delivery of the skin care composition onto an external surface of the skin. Processes for assembling absorbent articles such as the hemorrhoid treatment pads described herein include conventional techniques known in the art for constructing and configuring disposable absorbent articles. Suitable processes include joining topsheet and backsheet materials with an absorbent core interposed between the topsheet and backsheet, wherein the skin care composition is preferably applied on the topsheet. The terms "joined" and "joining" as used herein encompass configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element. For example, the backsheet and/or the topsheet can be joined to the absorbent core or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. Alternatively, the backsheet and/or topsheet can be joined to the absorbent core or to each other using heat bonding, pressure bonding, ultrasonic bonding, dynamic mechanical bonding, combinations of these attachment means, or any other suitable attachment means known in the art.

The topsheet, backsheet, and absorbent core are preferably joined such that the skin care compositions of the present invention are applied to the topsheet. It is contemplated, however, that the skin care composition can be applied to other areas of the absorbent article wherein these areas include wings, side panels, the absorbent core, any secondary layer intermediate the core and topsheet, or any other region of the absorbent article.

The skin care compositions of the present invention can also be delivered onto the skin by incorporating the compositions into aerosol dispensers, trigger spray dispensers, pump spray dispensers, jars, stick dispensers, cotton balls, patches, sponges, and any other type of known or otherwise effective delivery vehicle. The skin care composition can be applied in longitudinal stripes, transverse stripes, spots, or other non-continuous patterns. The skin care composition can also be applied continuously in a layer over the entire topsheet.

## EXAMPLES

The following examples further describe and demonstrate substances 46 suitable as skin care compositions within the scope of the present invention. The examples are given solely for the purpose of illustration and are not to be construed as limitations of the present invention, as many variations thereof are possible without departing from the scope of the invention. All exemplified concentrations are weight-weight percents, unless otherwise specified.

### Example I

The skin care compositions exemplified in Table 1 are representative of carrier systems of the skin care compositions. The carrier systems are generally prepared by combining, by weight, petrolatum and a fatty alcohol such as behenyl alcohol, and then heating the mixture while stirring to a temperature of about 80°C using a low speed propeller mixer. Next, viscosity or thickening agents are added to the mixture to shear mix the ingredients into a final carrier system. Suitable viscosity or thickening agents include beheneth-10, fumed silica, bentonite, and steareth-2, wherein the viscosity or thickening agents are used alone or in combination. The ingredients can be shear mixed at 11,000 revolutions per minute (rpm) using an IKA Ultra Turrax Shear Mixer.

Alternatively, the petrolatum, fatty alcohol, and viscosity or thickening agent can be combined, heated with stirring at 80°C to melt the ingredients, and then mixed into a final carrier system using a high speed blade mixer such as the Tokusyu Kika TK Robo Mics which operates at 5,000 rpm.

Table 1

| Carrier Systems | | | | | | | |
|---|---|---|---|---|---|---|---|
| **Component** | **Sample 1 (Wt. %)** | **Sample 2 (Wt. %)** | **Sample 3 (Wt. %)** | **Sample 4 (Wt. %)** | **Sample 5 (Wt. %)** | **Sample 6 (Wt. %)** | **Sample 7 (Wt. %)** |
| Petrolatum[1] | 78.1 | 67.8 | 70.0 | 70.0 | 70.0 | 73.5 | 85.0 |
| Behenyl Alcohol[2] | 8.7 | 29.0 | -- | 20.0 | 15.0 | 20.0 | 15.0 |
| Cetearyl Alcohol[3] | | | 30.0 | -- | -- | --- | --- |
| Beheneth-10[4] | 10.0 | -- | -- | -- | -- | --- | --- |
| Fumed Silica[5] | 3.2 | 3.2 | -- | -- | -- | --- | --- |
| Bentonite[6] | -- | -- | -- | 10.0 | -- | --- | --- |
| Steareth-2[7] | -- | -- | -- | -- | 15.0 | --- | --- |
| Span 60[8] | -- | -- | -- | -- | -- | 6.5 | --- |

Wt. % - weight percent
1 - petrolatum available as Protopet® 1S from the Witco Corporation
2 - behenyl alcohol available as Lanette 22 from the Cognis Corporation
3 - cetearyl alcohol available as Stenol 1822 from the Cognis Corporation
4 - beheneth-10 available as Mergital® B10 from the Cognis Corporation
5 - fumed silica available as Cabosil® TS-720 from the Cabot Corporation
6 - bentonite available as Bentone® 38 from the Rheox Incorporation
7 - steareth-2 available as Brij@ 762 from the Uniqema Corporation
8. sorbitan monostearate available as Span 60 from the Uniqema Corporation

**Examples II-IX**

The following Examples II-IX illustrated hereinbelow in Table 2 are representative of skin care compositions that include the carrier systems identified in Table 1. The skin care compositions are prepared by formulating a premix solution of the zinc oxide skin treatment agent and adding the zinc oxide premix to the other skin treatment agents and any optional ingredients such as panthenol and glycerin, or by formulating a skin treatment solution of hexamidine and niacinamide skin treatment agents and any optional ingredients. The skin treatment solution is then added to a carrier system such as those described in Table 1, wherein the skin treatment solution and carrier system is heated while stirring to a temperature of about 80°C. All ingredients are included by weight of the skin care compositions. These skin care compositions are especially effective in the control of skin disorders such as skin erythema, malodor, and skin bacterial infections.

Table 2

| Skin Care Compositions | | | | | | | | | |
|---|---|---|---|---|---|---|---|---|---|
| **Component** | **Ex. II (Wt.%)** | **Ex. III (Wt. %)** | **Ex. IV (Wt. %)** | **Ex. V (Wt. %)** | **Ex. VI (Wt. %)** | **Ex. VII (Wt. %)** | **Ex VIII (Wt. %)** | **Ex IX (Wt. %)** | **Ex X (Wt. %)** |
| Sample 1 | 97.1 | 89.8 | -- | -- | -- | -- | -- | | |
| Sample 2 | -- | -- | 96.2 | 99.7 | -- | -- | -- | | |
| Sample 3 | -- | -- | -- | -- | 95.7 | -- | -- | | |
| Sample 4 | -- | -- | -- | -- | -- | 97.3 | -- | | |
| Sample 5 | -- | -- | -- | -- | -- | -- | 97.8 | | |

(continued)

| Skin Care Compositions | | | | | | | | | |
|---|---|---|---|---|---|---|---|---|---|
| **Component** | **Ex. II (Wt.%)** | **Ex. III (Wt. %)** | **Ex. IV (Wt. %)** | **Ex. V (Wt. %)** | **Ex. VI (Wt. %)** | **Ex. VII (Wt. %)** | **Ex VIII (Wt. %)** | **Ex IX (Wt. %)** | **Ex X (Wt. %)** |
| Sample 6 | -- | -- | -- | -- | -- | -- | -- | 99.5 | |
| Sample 7 | -- | -- | -- | -- | -- | -- | --- | | 100 |
| ZnO Premix[9] | 0.7 | 7.1 | 0.75 | 0.2 | -- | -- | -- | | |
| Hexamidine[10] | 0.1 | 0.03 | 0.05 | 0.1 | 0.05 | 0.02 | 0.1 | | |
| Panthenol[11] | 0.5 | 0.5 | 0.5 | -- | 0.5 | 0.25 | -- | | |
| Glycerine[12] | 0.1 | -- | -- | -- | -- | -- | 0.1 | | |
| Niacinamide[13] | 1.0 | 2.0 | 2.0 | -- | -- | -- | 2.0 | | |
| Acidified Niacinamide[14] | -- | -- | -- | -- | 3.7 | 1.9 | -- | | |
| Chamomile[15] | 0.5 | --- | 0.5 | -- | -- | 0.5 | -- | 0.5 | --- |

9 - Zinc oxide premix comprising 70% zinc oxide mixture of ULTRAFINE 350 zinc oxide available from the Kobo Incorporation, Arlecel® P100 available from the Uniqema Incorporation, and Salacos® 99 available from the Ikeda Incorporation
10 - hexamidine available as hexamidine diisethionate from Laboratories Serolobilogiques under the tradename ELASTAB HP 100
11 - panthenol available as D-panthenol from Roche Vitamins Incorporation
12 - glycerine available as Glycerine, USP Kosher® from the Procter & Gamble Company
13 - niacinamide available from Em Industries HHN
14 - acidified niacinamide made by reacting niacinamide with stearic acid
15 - chamomile available as Phytoconcentrol Chamomile from Dragoco

The skin care composition of Example II can be subsequently applied by spraying the composition onto the entire body surface wearer-contacting surface of the hemorrhoid treatment pad 20. To deliver a safe and effective amount of the skin care composition onto the skin, about 30 g/$m^2$ (19.4 mg/$in^2$) of the skin care composition is applied to the topsheet using a hot melt pneumatic Dynatec E84B1758 spray head having a head operating temperature of about 90°C and an atomization pressure of about 16 kiloPascals (kPa).

The skin care composition of Example III can be subsequently applied to a topsheet component of the hemorrhoid treatment pad 20 using a contact applicator such as a Nordsen EP 11-12-02. The skin care composition can be applied as alternating stripes having 4 mm width. The topsheet is then assembled, with the composition on the body side surface, to form a hemorrhoid treatment pad 20. To deliver a safe and effective amount of the skin care composition onto the skin, about 155 g/$m^2$ (100 mg/$in^2$) of the skin care composition is applied to the topsheet.

The skin care composition of Example IV can be subsequently applied to a topsheet component of the hemorrhoid treatment pad 20 using a contact applicator such as a Nordsen EP 11-12-02. The topsheet is then assembled, with the composition on the body side surface, to form a hemorrhoid treatment pad 20. To deliver a safe and effective amount of the skin care composition onto the skin, about 0.5 g/$m^2$ (0.32 mg/$in^2$) of the skin care composition is applied to the topsheet.

The skin care composition of Example V can be subsequently applied by spraying the composition onto the entire body surface wearer-contacting surface of the hemorrhoid treatment pad 20. To deliver a safe and effective amount of the skin care composition onto the skin, about 60 g/$m^2$ (38.7 mg/$in^2$) of the skin care composition is applied to the topsheet using a hot melt pneumatic Dynatec E84B1758 spray head having a head operating temperature of about 90°C and an atomization pressure of about 16 kiloPascals (kPa).

The skin care composition of Example VI can be subsequently applied to a topsheet component of the hemorrhoid treatment pad 20 using a contact applicator such as a Nordsen EP 11-12-02. The skin care composition is applied as alternating stripes having 5 mm width. The topsheet is then assembled, with the composition on the body side surface, to form a hemorrhoid treatment pad 20. To deliver a safe and effective amount of the skin care composition onto the skin, about 31 g/$m^2$ (20 mg/$in^2$) of the skin care composition is applied to the topsheet.

The skin care composition of Example VII can be subsequently applied to a topsheet component of the hemorrhoid treatment pad 20 using a contact applicator such as a Nordsen EP 11-12-02. The skin care composition is applied as alternating stripes having 3mm width. The topsheet is then assembled, with the composition on the body side surface, to form a hemorrhoid treatment pad 20. To deliver a safe and effective amount of the skin care composition onto the skin, about 5 g/$m^2$ (3.2 mg/$in^2$) of the skin care composition is applied to the topsheet.

The skin care composition of Example VIII can be subsequently applied by spraying the composition onto the entire body surface wearer-contacting surface of the hemorrhoid treatment pad 20. To deliver a safe and effective amount of the skin care composition onto the skin, about 310 g/$m^2$ (200 mg/$in^2$) of the skin care composition is applied to the topsheet using a hot melt pneumatic Dynatec E84B1758 spray head having a head operating temperature of about 90°C and an atomization pressure of about 16 kiloPascals (kPa).

The skin care composition of Example IX can be subsequently applied by slot coating (Nordsen EP 11-12-02) striped configurations of the composition onto the wearer-contacting surface of a hydrophobic spunbond bicomponent polyethylene / polypropylene topsheet (BBA, Washougal, WA) of the hemorrhoid treatment pad 20. To deliver a safe and effective amount of the lotion composition onto the skin, the lotion composition is applied to the topsheet in a striped configuration wherein the striped configuration comprises at least two stripes each being 5 millimeters (mm) wide x 60 mm long and having about 20 g/$cm^2$ (12.9 mg/$in^2$) of the composition applied thereon. The topsheet is then assembled, with the composition on the body side surface, to form a hemorrhoid treatment pad 20.

The skin care composition of Example X can be subsequently applied by spraying the composition onto the entire body surface wearer-contacting surface of the hemorrhoid treatment pad 20. To deliver a safe and effective amount of the skin care composition onto the skin, about 3.3 g/$m^2$ (2.13 mg/$in^2$) of the skin care composition is applied to the topsheet using a hot melt pneumatic Dynatec E84B1758 spray head having a head operating temperature of about 90°C and an atomization pressure of about 16 kiloPascals (kPa).

## Test Methods

Unless otherwise noted, all of the methods are conducted under the standard laboratory climatic control as specified by TAPPI under T402 om-93, Section 3 (73°F or 23 °C ± 1 °C, 50% ± 2% RH). All samples should be acclimated to these conditions for two hours prior to measurements.

## Absorbent Capacity Test

The article is weighed to the nearest 0.1 gram. The article is then submerged in a beaker of sterile 0.9% saline solution (obtainable from the Baxter Travenol Company of Deerfield, IL), such that the article is totally submerged and is not bent or otherwise twisted or folded. The article is submerged for 10 minutes. The article is removed from the saline and laid horizontally on a wire mesh screen having square openings 0.25 inches by 0.25 inches (0.64 cm by 0.64 cm) for five minutes to allow the saline to drain out to the article. Both sides of the article are then covered with absorbent blotters, such as the filter paper #631 available from the Filtration Science Corp., Eaton-Dikeman Division of Mount Holly Springs, PA. A uniform 1 pound per square inch (6.9 Pa) load is placed over the article to squeeze excess fluid out. The absorbent blotters are replaced every 30 seconds until the amount of fluid transferred to the absorbent blotters is less than 0.5 grams in a 30 second period. Next, the article is weighed to the nearest 0.1 gram and the dry weight of the article is subtracted. The difference in grams is the absorbent capacity of the article.

## Topsheet Compressibility (Thickness Change) Test

This test procedure determines the topsheet caliper changes under pressures. This procedure can be used to calculate topsheet compressibility (and density) as a functions of two applied pressure loads. These pressures may be typical of those found within the natal cleft while wearing a hemorrhoid treatment pad. However, this procedure is intended to be used to test topsheet materials whenever topsheet compressibility is specified in the appended claims, and is not limited to testing topsheets used only on hemorrhoid treatment products.

### Apparatus:

This testing procedure requires an accurate (to +/- 0.01 mm) thickness gauge such as a Ono-Sokki GS-503 gauge available from Ono-Sokki Technology, Inc. of Addison, IL, USA equipped with an interchangeable circular foot. Ideally, the thickness gauge is attached to a digital readout module, such as a Ono-Sokki model DG-3610. To ensure no meaningful irregularities are present on the surface upon which samples are to be placed and measured, that would otherwise lead to measurement errors, a smoothly polished granite block (such as supplied by Rock of Ages Surface Plate, Barre, Vermont, USA polished to at least 0.001 mm flatness) or equivalent should be used as the measurement surface.

Circular measurement foot:

| | |
|---|---|
| A circular flat foot of diameter | = 40.0 mm ± 0.5 mm and a weight of 10 g has been chosen. |
| Complete weight (foot + caliper shaft) | = 32g ± 1g. |
| Initial measurement pressure | = 250 $N/m^2$ (2.55 $g/cm^2$ or 0.036 psi) |
| Additional weight attached to shaft | = 100 g ± 1g |
| Final weight (foot + weight + shaft) | = 132 g ± 1g |
| Final measurement pressure | = 1000 $N/m^2$ (10.5 $g/cm^2$ or 0.149 psi) |

Pad samples should be cut such that they are larger than the 40 mm diameter circular foot that is used to compress the sample. A rectangular cutting die 10 cm by 10 cm was found to work well and to ensure no edge effects associated with irrecoverable sample compression during cutting occur. The area to be tested should be able to lie flat (preferably, less than 0.05 mm deviation in flatness). The structure of the topsheet should not, however, be altered in order to flatten the samples. Samples may be cut from a raw material roll or a finished product but the cut area must be free from wrinkles or curvature and must be representative of the undistorted dimensions of the topsheet material. The topsheet layer must be cleanly separable from other materials. At least three different samples should be measured with the average result reported.

For topsheet samples that are removed from a finished product and if the sample dimensions are not large enough to measure as described above, then a smaller diameter circular foot will need to be chosen. Provided that the applied pressure is maintained, as detailed below, and irregular edges or material damage linked to topsheet removal are avoided, the use of a smaller diameter circular foot is acceptable. If a smaller diameter foot is utilized, then a minimum of five samples should be measured to ensure a representative measurement of the topsheet surface is obtained. Ensure the topsheet is carefully removed without alteration of its surface structure.

Procedure:

1. Ensure the caliper gauge is calibrated using a standard gauge prior to commencing the measurements and accurate to ± 0.01 mm.

2. Place the sample with body facing surface facing upwards on the polished granite surface with the caliper shaft and foot assembly unit positioned centrally above the sample to be tested. The test is commenced with a standard circular foot (40 mm) and a total shaft+foot weight of 32 g.

3. Gently lower the caliper shaft and foot assembly onto the sample and after waiting 5 seconds (but not more than 10 seconds) record initial sample thickness to the nearest 0.01 mm. This corresponds to the thickness under a pressure of 250 $N/m^2$ (2.55 $g/cm^2$ or 0.036 psi).

4. With the sample still positioned under the circular foot carefully place the additional weight disc (100 g) onto the caliper shaft (the total weight is now 132 g) and after waiting 5 seconds (but not more than 10 seconds) record the final sample thickness. This corresponds to the thickness under a pressure of 1000 $N/m^2$ (10.5 $g/cm^2$ or 0.149 psi).

5. The change in topsheet caliper is determined by subtracting the final caliper from the initial caliper (see calculation below).

Calculations:

The change in topsheet caliper as a result of compressive forces is determined by;

$$\text{Topsheet Caliper Change} = \text{Caliper (at 250 } N/m^2) - \text{Caliper } (1000\ N/m^2)$$

The greater the Topsheet Caliper Change number, the more the topsheet will be capable of compressing and

adapting to compressive forces during wear of the absorbent device without directly passing these forces on to the sensitive membranes of the wearer's body.

**Water Dispersion Test**

Apparatus

| | |
|---|---|
| Shaker | Junior Orbit Shaker available from Lab Line Instruments of Melrose Park, Illinois. |
| Thermometer | 30 to 120°F with 1 degree divisions |
| Timer | Digital stopwatch |
| Jar with Lid | 16 oz. glass jar with lid. |

Test Setup

1. Fill the glass jar with 300 ml. of 73±3°F tap water.
2. Set the speed on the Junior Orbit Shaker to 250 rpm according to the manufacturer's directions.

Procedure

1. Hold a sample (e.g. an absorbent interlabial device 20) 3 to 4 inches (7.6 to 10.2 centimeters) above the surface of the water in the jar. Gently drop the sample onto the water surface.

2. Place the lid on the jar.

3. Place the jar into the Junior Orbit Shaker such that the jar is oriented on its side.

4. Start the Junior Orbit shaker with the on/off switch, starting the timer when the shaker is turned on.

5. Record the time required until the sample separates into at least two pieces. Separation does not include the disassociation of a few individual fibers from an otherwise intact sample. The time is the total time the sample is being shaken.

6. Repeat steps 1 through 5 with three additional samples.

Calculation and Reporting

Calculate and report the mean and standard deviation of the water dispersibility time for the four samples tested.

**Flushability Test**

Overview

As noted above, the terms "flushable or flushability" refer to a product's capacity to pass through typical commercially available household toilets and plumbing drainage systems without causing clogging or similar problems that can be directly associated with the physical characteristics of the product. For the purpose of the appended claims, the products are evaluated for flushability via relative ease of toilet bowl and trap evacuation and subsequent transport through a simulated plumbing system. The flushability of such a device should be measured by the following test procedure.

The test procedure is designed to simulate two days of normal toilet usage for a family of 4 (2 men, 2 women). The test employs a flushing sequence to simulate the following conditions: male urination visits, female urination visits (including post urinary drying with tissue), disposal of the product (that is, the interlabial device or other device to be

tested) with cleaning using tissue, and bowel movement visits. The amount of tissue to be used for each tissue flush is a normal loading of 2 strips of seven sheets. The normal lading is based on consumer research regarding typical habits and practices. The test is designed to simulate the conditions a product will encounter if it is flushed through a conventional toilet and into a municipal sewer or into a septic tank. Samples are evaluated for: 1) toilet bowl and trap clearance, 2) drain line blockage, and 3) disintegration during flushing.

Apparatus

An apparatus suitable for the flushability test is shown in plan view in FIG. 5. The apparatus includes:

- a 3.5 gallon (13.2 liter) water saver siphon vortex toilet referred to as 210 (additional toilets can also be attached to the piping layout shown in FIG. 5 to evaluate the behavior of test samples using different flushing mechanisms such as commercial, pressure toilets);
- approximately 59 feet (18 meters) of 4 inch (10 cm) inside diameter acrylic pipe (As can be seen from FIG. 5, the piping is assembled in roughly a square configuration having linear runs 211, 213, 215, 217, 219, 221 approximately 10 feet (3 meters) long);
- a cast iron tee 223 slightly downstream of the toilet 210 that is open to the atmosphere for venting;
- five cast iron ninety degree elbows 212, 214, 216, 218, and 220;
- a snag 222 positioned vertically (FIG. 6) approximately 15 feet from the pipe's terminal end and approximately 1 inch (2.5 cm) long; and
- a screen (No. 4 Tyler sieve) to capture solid effluent for evaluation of disintegration.

The apparatus used for this method is set up to be equivalent to ANSI Standard A112.19.2M-1990 for Vitreous China fixtures. The piping is plumbed to provide a drop of 0.25 inch per foot (2 centimeters/meter) of pipe length.

Materials

Tissue Product used in Test: standard "CHARMIN" toilet tissue manufactured by The Procter & Gamble Company of Cincinnati, Ohio.
Synthetic Fecal Material: Prepared according to the method described below

Test Flushing Sequence

The test flushing sequence simulates 2 days of normal toilet usage for a family of 4 (2 men, 2 women; based on consumer habits and practices research). The sequence of 34 total flushes consists of 14 flushes with an empty bowl, 8 flushes with tissue only, 6 flushes with tissue and the product to be tested and 6 flushes with tissue and simulated fecal matter (SFM). When it is used, the SFM is placed in the bowl just prior to the addition of tissue. The SFM loading of 160 g $\pm$ 5 g consists of two 1 inch (2.5 centimeter) x 4 inch (10 centimeter) pieces and one 1 inch (2.5 centimeter) x 2 inch (5 centimeter) piece. Folded tissue strips (or the catamenial product) are placed in the bowl at 10 second intervals. Ten seconds after the final strip or product is placed into the bowl, the toilet is flushed. The flushing sequence is described below as a series of two routines combined in the following order:

Routine #1 (To be performed first 6 times for a total of 30 flushes)

1) Flush With Tissue Only - Take a drain line blockage reading 2 minutes after the water reaches the simulated obstruction, wait 1 additional minute, and move to step 2.

2) Flush With Empty Bowl. Take a drain line blockage reading 2 minutes after the water reaches the snag point and move to step 3.

3) Flush With Tissue and Product - Take a drain line blockage reading 2 minutes after the water reaches the

snag point, wait 1 additional minute, and move to step 4.

4) Flush With Empty Bowl. Take a drain line blockage reading 2 minutes after the water reaches the snag point and move to step 5.

5) Flush With Tissue and Simulated Fecal Matter (SFM). Take a drain line blockage reading 2 minutes after the water reaches the snag point, wait 1 additional minute.

Routine #2 (To be performed 1 time)

1) Flush With Tissue Only - Take a drain line blockage reading 2 minutes after the water reaches the snag point, wait 1 additional minute, and move to step 2.

2) Flush With Empty Bowl. Take a drain line blockage reading 2 minutes after the water reaches the snag point and move to step 3.

3) Flush With Tissue Only - Take a drain line blockage reading 2 minutes after the water reaches the snag point, wait 1 additional minute, and move to step 4.

4) Flush With Empty Bowl. Take a drain line blockage reading 2 minutes after the water reaches the snag point.

Total number of flushes per sequence is 34.

If, after the second flush in the flushing sequence, the product remains in the bowl or trap after flushing, the tissue and or product is plunged into the drainage line manually and the flushing sequence will continue. After completion of each trial loading, the drainage pipe will be cleared prior to beginning subsequent testing.

The above described flushing sequence is repeated three times for each test product.

Data Reporting

The degree of drain line blockage is determined by measuring the length of water dammed up behind the obstruction. Graduations are marked every 12 inches (30 centimeters) on the drainpipe upstream of the obstruction. Each one foot length that the water is backed up corresponds to 0.25 inch (0.6 centimeter) or 6.25% of blockage at the obstruction point. Test product residues which exit the drainpipe are also collected.

The following data are recorded for each evaluation:

1) Incidence of failure (%) of the product to clear bowl and trap in one flush

2) Incidence of failure (%) of the product to clear bowl and trap in two flushes

3) Incidence of product on simulated snag

4) Maximum level (%) of drain line blockage

5) Cumulative level (%) of drain line blockage over the 2 day simulated test period.

By flushable as used herein is meant that the products described herein will completely clear the bowl at least about 70% of the time in two or fewer flushes, more preferably at least about 80% of the time in one flush, even more preferably at least about 90% of the time in one flush, and most preferably at least about 95% of the time in one flush. The products described herein will preferably have a maximum level of drain line blockage of less than or equal to about 80%. The products described herein will preferably have a cumulative level of drain line blockage over the 2 day simulated test period of less than or equal to about 50%.

**28 Day Sludge Test**

Purpose:

To determine the extent to which an absorbent article disintegrates upon exposure to biologically active anaerobic sludge. Anaerobic conditions are typically found in household septic tanks, as well as in municipal sewage treatment facilities in the form of anaerobic sludge digesters. Test products, such as the absorbent article are combined with anaerobic digester sludge to determine the extent and rate of disintegration of test products over a 28 day period. Disintegration (as measured by weight change) is typically measured on days 3, 7 14, 21 and 28 of the particular study. This protocol is modeled after the National Sanitation Foundation, Ann Arbor, Michigan, International Protocol: Evaluation of the Anaerobic Disintegration of a Test Product, November, 1992.

Materials:

Control Product

TAMPAX Regular brand tampons will be used as a positive control product in the anaerobic disintegration test.

Material Preparation

Prior to the addition of the test and control products to the reactors, the materials will be dried in a hot air oven at 103° ± 2°C for 2 hours and then weighed to determine the initial weight. Approximately equal weights of the control and the test products will be placed in respective reactors.

Anaerobic sludge:

The sludge used in this evaluation will be anaerobic sludge obtained from a municipal waste water treatment plant, or raw sewage obtained as influent from a waste water treatment plant that has been concentrated by settling and decanting the overlying water. Prior to use in the evaluation, the following parameters of the sludge will be measured in accordance with standard laboratory operating procedures:

Total solids

Total volatile solids

pH

The sludge should meet the following criteria for use in the evaluation:

pH between 6.5 and 8

Total solids ≥ 15,000 mg/L

Total volatile solids ≥ 10,000 mg/L

The criteria for the activity of the sludge requires that the control tampon material must lose at least 95% of its initial dry weight after 28 days exposure.

Procedure:

The test and control products are added to a 2L wide mouth glass flask (reactor) containing 1500 ml of anaerobic digester sludge or concentrated raw sewage. Three reactor flasks per test material per sampling day are prepared. Thus, if disintegration is measured on days 3, 7, 14, 21, and 28, there will be a total of 15 reactor flasks for the test product and 15 flasks for the control product The reactors are sealed and placed in an incubator maintained at 35 ± 2°C. On the specified sampling days, three reactors each for the test and control material are removed from the incubator. On the designated sample days, the contents of each reactor will be passed through a 1mm mesh screen to recover any undisintegrated material. Any collected material will be rinsed with tap water, removed from the screen and placed in a hot air oven at 103 ± 2°C for at least 2 hours. The dried material will be weighed to determine final weight. Visual observations of the physical appearance of the materials when recovered from the reactors will also be made and recorded.

Results:

The rate and extent of anaerobic disintegration of each test material and the control material is determined from initial dry weights of the material and the dried weights of the material recovered on the sampling days. The percent anaerobic disintegration is determined using the following equation (percent weight loss):

$$\text{Percent Disintegration} = \frac{(\text{initial dry weight - final dry weight})}{(\text{initial dry weight})} \times 100$$

The average percent disintegration for the test and control products for each sampling day will be presented. For the purposes of the appended claims, the percent disintegration values are for day 28 of the study.

## Claims

1. A hemorrhoid treatment pad **characterized by** a skin care composition, the skin care composition comprising:

   (a) from 0.001 % to 0.1% by weight of hexamidine;
   (b) from 0.001 % to 10% by weight of zinc oxide;
   (c) from 0.01% to 10% by weight of niacinamide; and
   (d) a carrier.

2. The hemorrhoid treatment pad of Claim 1, wherein the composition comprises from 0.01% to 0.05% by weight of hexamidine, from 0.01% to 1% by weight of zinc oxide, and from 0.2% to 2% by weight of niacinamide.

3. The hemorrhoid treatment pad of Claim 1, wherein the hexamidine is selected from the group consisting of hexamidine isethionate, hexamidine diisethionate, hexamidine hydrochloride, hexamidine gluconate, and mixtures thereof.

4. The hemorrhoid treatment pad of Claim 1, wherein the composition further comprises herbs.

5. An absorbent anal pad intended to be inserted into the space defined by the opposing halves of the buttocks and adjacent the anus, said absorbent pad comprising a liquid pervious body contacting surface and an underside, said absorbent pad further comprising an axis for bending the pad along a central longitudinal axis, wherein said liquid pervious body contacting surface is joined to an absorbent material, wherein the anal pad has a skin care composition applied to said liquid pervious body contacting surface, and wherein the absorbent anal pad is folded in use along said central longitudinal axis for placement of said liquid pervious top surface adjacent to the anus, and wherein said and pad is a hemorrhoid treatment pad according to claim 1.

6. The absorbent anal pad of Claim 5, wherein said pad comprises an integral topsheet and core.

7. The absorbent anal pad of Claim 5, wherein said skin care composition is applied in an intermittent pattern over the body contacting surface of said pad.

8. The hemorrhoid treatment pad of Claim 7, wherein said intermittent pattern comprises at least one stripe of skin care composition.

## Patentansprüche

1. Einlage zur Hämorrhoidenbehandlung, **gekennzeichnet durch** eine Hautpflegezusammensetzung, wobei die Hautpflegezusammensetzung Folgendes umfasst:

   (a) von 0,001 Gew.-% bis 0,1 Gew.-% Hexamidin;
   (b) von 0,001 Gew.-% bis 10 Gew.-% Zinkoxid;

(c) von 0,01 Gew.-% bis 10 Gew.-% Niacinamid; und
(d) einen Träger.

2. Einlage zur Hämorrhoidenbehandlung nach Anspruch 1, wobei die Zusammensetzung von 0,01 Gew.-% bis 0,05 Gew.-% Hexamidin, von 0,01 Gew.-% bis 1 Gew.-% Zinkoxid und von 0,2 Gew.-% bis 2 Gew.-% Niacinamid umfasst.

3. Einlage zur Hämorrhoidenbehandlung nach Anspruch 1, wobei das Hexamidin ausgewählt ist aus der Gruppe bestehend aus Hexamidinisethionat, Hexamidindüsethionat, Hexamidinhydrochlorid, Hexamidingluconat und Mischungen davon.

4. Einlage zur Hämorrhoidenbehandlung nach Anspruch 1, wobei die Zusammensetzung ferner Kräuter umfasst.

5. Absorptionsanaleinlage zum Einführen in den Raum, der durch die gegenüberliegenden Gesäßhälften bestimmt wird und an den Anus angrenzt, wobei die Absorptionseinlage eine flüssigkeitsdurchlässige Körperkontaktoberfläche und eine Unterseite umfasst, wobei die Absorptionseinlage ferner eine Achse zum Biegen der Einlage entlang einer Mittellängsachse umfasst, wobei die flüssigkeitsdwchlässige Körperkontaktoberfläche mit einem Absorptionsmaterial verbunden ist, wobei die Analeinlage eine auf die flüssigkeitsdurchlässige Körperkontaktoberfläche aufgetragene Hautpflegezusammensetzung aufweist und wobei die Absorptionsanaleinlage bei Gebrauch entlang der Mittellängsachse gefaltet wird, um die flüssigkeitsdurchlässige obere Oberfläche an den Anus anliegend zu platzieren, und wobei die Analeinlage eine Einlage zur Hämorrhoidenbehandlung nach Anspruch 1 ist.

6. Absorptionsanaleinlage nach Anspruch 5, wobei die Einlage eine integrale Oberschicht und einen integralen Kern umfasst.

7. Absorptionsanaleinlage nach Anspruch 5, wobei die Hautpflegezusammensetzung in einem intermittierenden Muster über der Körperkontaktoberfläche der Einlage aufgetragen wird.

8. Einlage zur Hämorrhoidenbehandlung nach Anspruch 7, wobei das intermittierende Muster mindestens einen Streifen der Hautpflegezusammensetzung umfasst.

**Revendications**

1. Tampon de traitement des hémorroïdes **caractérisé par** une composition de soin de la peau, la composition de soin de la peau comprenant :

(a) de 0,001 % à 0,1 % en poids d'hexamidine ;
(b) de 0,001 % à 10 % en poids d'oxyde de zinc ;
(c) de 0,01 % à 10 % en poids de niacinamide ; et
(d) un véhicule.

2. Tampon de traitement des hémorroïdes selon la revendication 1, dans lequel la composition comprend de 0,01 % à 0,05 % en poids d'hexamidine, de 0,01 % à 1 % en poids d'oxyde de zinc, et de 0,2 % à 2 % en poids de niacinamide.

3. Tampon de traitement des hémorroïdes selon la revendication 1, dans lequel l'hexamidine est choisie dans le groupe constitué d'iséthionate d'hexamidine, di-iséthionate d'hexamidine, chlorhydrate d'hexamidine, gluconate d'hexamidine, et leurs mélanges.

4. Tampon de traitement des hémorroïdes selon la revendication 1, dans lequel la composition comprend en outre des herbes.

5. Tampon anal absorbant prévu pour être inséré dans l'espace défini par les moitiés opposées des fesses et de façon adjacente à l'anus, ledit tampon absorbant comprenant une surface en contact avec le corps perméable aux liquides et une surface inférieure, ledit tampon absorbant comprenant, en outre, un axe pour plier le tampon le long d'un axe longitudinal central, dans lequel ladite surface en contact avec le corps perméable aux liquides est jointe à un matériau absorbant, où le tampon anal comporte une composition de soin de la peau appliquée à ladite surface en contact avec le corps perméable aux liquides, et où le tampon anal absorbant est plié en cours d'utilisation le long dudit axe longitudinal central pour la mise en place de ladite surface supérieure perméable aux liquides de façon

adjacente à l'anus, et où ledit tampon anal est un tampon de traitement des hémorroïdes selon la revendication 1.

**6.** Tampon anal absorbant selon la revendication 5, où ledit tampon comprend une feuille de dessus et une âme d'un seul tenant.

**7.** Tampon anal absorbant selon la revendication 5, dans lequel ladite composition de soin de la peau est appliquée dans un motif intermittent sur la surface en contact avec le corps dudit tampon.

**8.** Tampon de traitement des hémorroïdes selon la revendication 7, dans lequel ledit motif intermittent comprend au moins une bande de composition de soin de la peau.

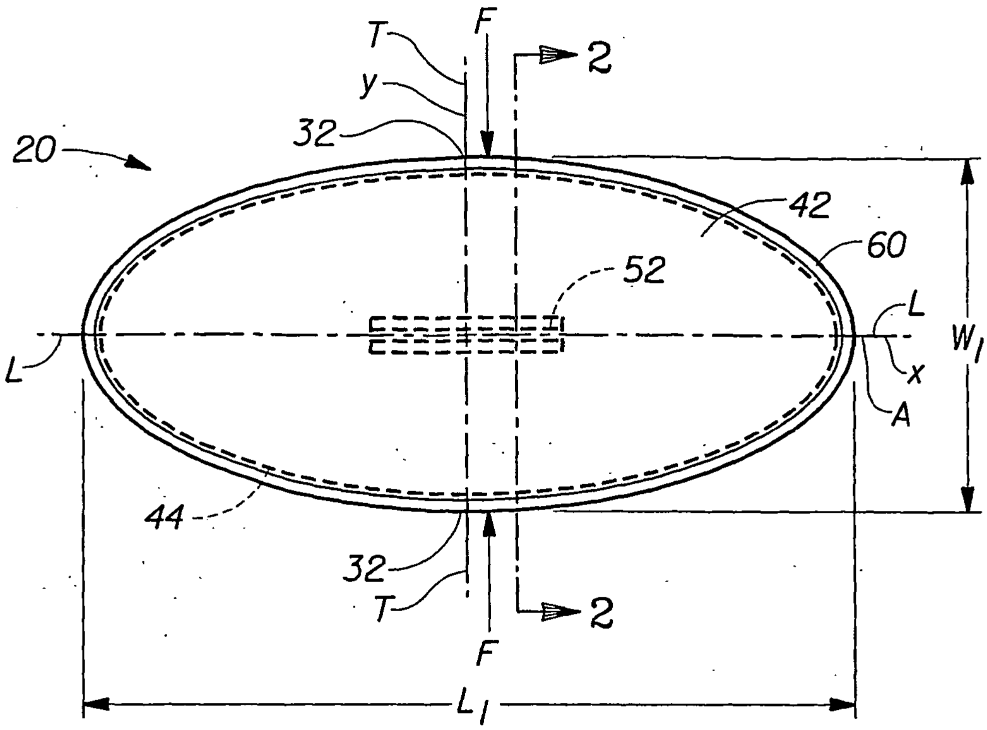


Fig. 1

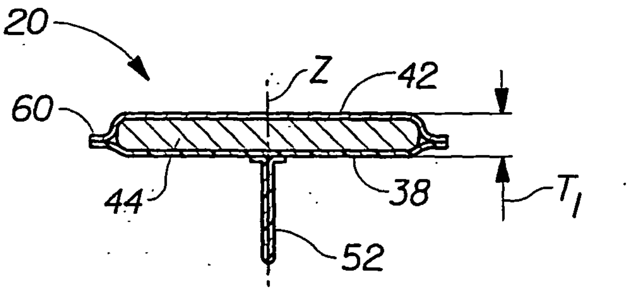


Fig. 2

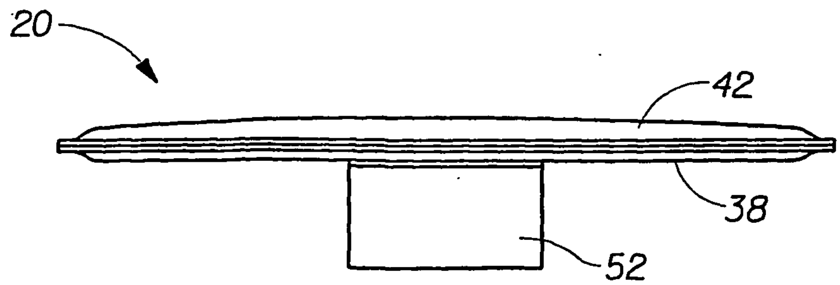


Fig. 3

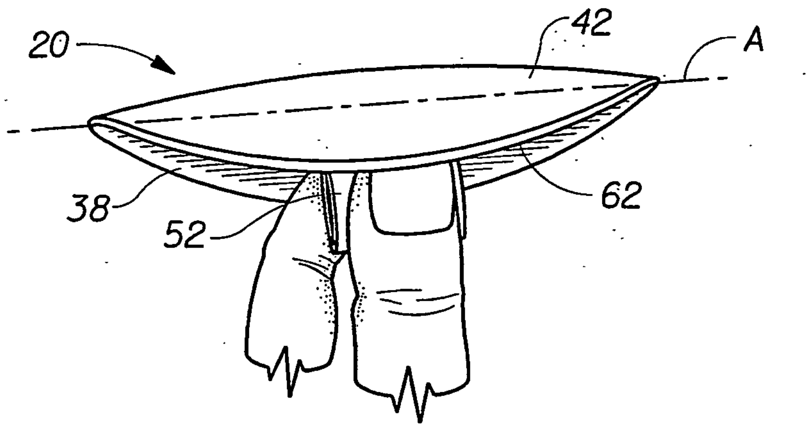


Fig. 4

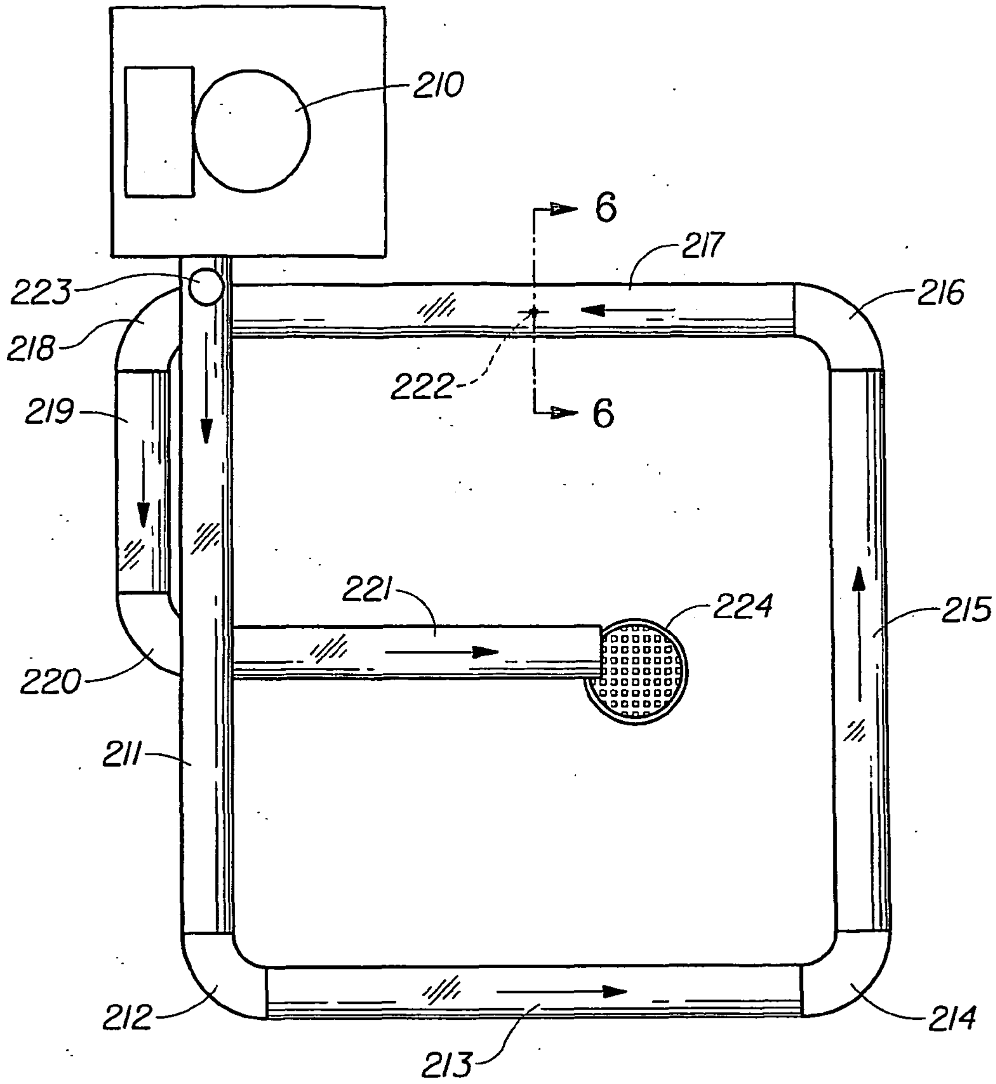
210
6
217
223
216
218
222
6
219
221
224
215
220
211
212
213
214

Fig. 5

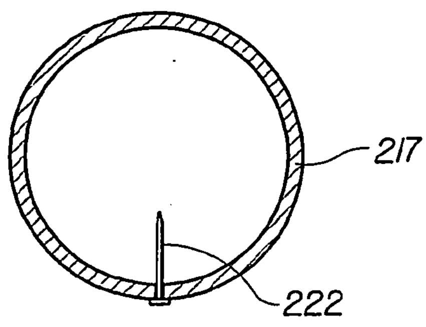
217
222

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 2742042 A
- US RE24385 E, Flanders
- US 3570489 A, Brown
- US 4484919 A, Sohn
- US 5695484 A, Cox
- US 4702237 A, Gianopoulos
- US 20010003157 A1, Toth
- WO 0002508 A, Gomez
- US 5085884 A
- US 5422131 A, Young
- US 5429628 A, Trihn
- US 5780020 A, Peterson
- US 5518801 A, Chappell
- US 4609518 A
- US 4629643 A, Curro
- US 637440 A
- US 09905804 A
- WO 0213750 A
- US 3929135 A
- US 4777073 A, Exxon's
- US 5376655 A, Imaki
- US 5509282 A, Ampulski
- US 6153209 A, Vega
- WO 9945973 A, Donald C. Roe
- EP 111202 A, Nordsen

### Non-patent literature cited in the description

- The Merck Index. 1989, 1599
- Cosmetics, Science and Technology. 1972, vol. 1, 27-104
- The Merck Index. 1989, 464